(19) Europäisches Patentamt
European Patent Office
Office européen des brevets

(11) **EP 4 342 993 A1**

(12) # EUROPEAN PATENT APPLICATION
published in accordance with Art. 153(4) EPC

(43) Date of publication:
**27.03.2024 Bulletin 2024/13**

(21) Application number: **22804725.4**

(22) Date of filing: **18.05.2022**

(51) International Patent Classification (IPC):
***C12N 15/88*** *(2006.01)* ***A61K 9/10*** *(2006.01)*
***A61K 9/14*** *(2006.01)* ***A61K 31/7088*** *(2006.01)*
***A61K 31/7115*** *(2006.01)* ***A61K 39/12*** *(2006.01)*
***A61K 47/18*** *(2017.01)* ***A61K 47/24*** *(2006.01)*
***A61K 47/28*** *(2006.01)* ***A61K 47/34*** *(2017.01)*
***A61K 48/00*** *(2006.01)* ***A61P 11/00*** *(2006.01)*
***A61P 15/00*** *(2006.01)* ***A61P 31/20*** *(2006.01)*
***A61P 37/04*** *(2006.01)* ***C12N 15/34*** *(2006.01)*

(52) Cooperative Patent Classification (CPC):
**A61K 9/10; A61K 9/14; A61K 31/7088; A61K 31/7115; A61K 39/12; A61K 47/18; A61K 47/24; A61K 47/28; A61K 47/34; A61K 48/00; A61P 11/00; A61P 15/00; A61P 31/20; A61P 37/04; C07K 14/01;** (Cont.)

(86) International application number:
**PCT/JP2022/020713**

(87) International publication number:
**WO 2022/244815 (24.11.2022 Gazette 2022/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.05.2021 JP 2021084441**

(71) Applicant: **Daiichi Sankyo Company, Limited**
**Tokyo 103-8426 (JP)**

(72) Inventors:
- **ONODERA, Yoshikuni**
  **Tokyo 103-8426 (JP)**
- **TANOUE, Sumika**
  **Tokyo 103-8426 (JP)**
- **NIWA, Takako**
  **Tokyo 103-8426 (JP)**
- **KOIZUMI, Makoto**
  **Tokyo 103-8426 (JP)**

(74) Representative: **Vossius & Partner**
**Patentanwälte Rechtsanwälte mbB**
**Siebertstrasse 3**
**81675 München (DE)**

Remarks:
The complete document including Reference Table(s) and the Sequence Listing(s) can be downloaded from the EPO website

(54) **HPV INFECTIOUS DISEASE VACCINE**

(57) Provided are lipid particles encapsulating a nucleic acid capable of expressing an E6 antigen and an E7 antigen of human papillomavirus, whereby a vaccine for preventing and/or treating infection with human papillomavirus type 6 and/or type 11 can be provided. The lipid particles comprise a lipid that is a cationic lipid having the general formula (Ia), or a pharmaceutically acceptable salt thereof.

EP 4 342 993 A1

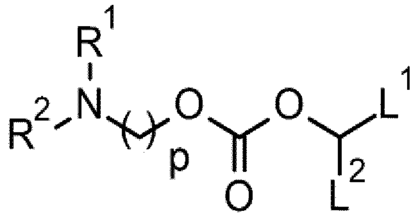

(Ia)

[In the formula, $R^1$, $R^2$, p, $L^1$ and $L^2$ are as defined in the specification.]

(52) Cooperative Patent Classification (CPC): (Cont.)
**C12N 15/88**

## Description

### Technical Field

The present invention relates to a nucleic acid lipid particle vaccine encapsulating mRNA encoding the antigens of human papillomavirus (HPV) type 6 and/or type 11.

### Background Art

Human papillomavirus (HPV) is a virus having circular double-stranded DNA as a genome without envelope membrane, and has about 200 genotypes (Non Patent Literature 1). Some of the genotypes turn infected cells cancerous, and in particular, genotypes 16 and 18, which have been demonstrated to correlate with onset of cancers, including uterine cervical cancer, are classified as high-risk types (Non Patent Literature 2). On the other hand, most other genotypes, such as genotypes 6 and 11, cause epithelial hyperplasia, which is benign and rarely leads to a malignant tumor, and therefore these genotypes are classified as low-risk types (Non Patent Literature 3).

In the genome of HPV, eight viral proteins are encoded, which are classified into early genes (E1, E2, E4, E5, E6 and E7) and late genes (L1 and L2) depending on the time of expression in the lifecycle of the virus. The early genes control virus replication and canceration of infected cells, and L1 and L2 are structural proteins forming outer shells (capsids) of virus particles (Non Patent Literature 4).

Virus proteins E6 and E7 in high-risk type HPV cause abnormal cell proliferation of infected cells. This is because the functions of p53, engaged in induction of apoptotic cell death, and pRb, one of Rb family proteins having a cell cycle regulating function, are inhibited by E6 and E7 (Non Patent Literatures 5 and 6). Even for low-risk type HPV, it has been suggested that p53 is inhibited by E6, and p130, which performs a cell cycle regulating function mainly on the epithelial side, is inhibited by E7 (Non Patent Literature 3). Furthermore, regions important for canceration activity of E6 and E7 have been revealed, and in the case where E6 and E7 are used as vaccine antigens, an inactivating mutation can be inserted to enhance safety (Non Patent Literatures 7 to 9).

The HPV genotypes 6 and 11 infect basal cells through wounded regions in the mucosal epithelia of the respiratory tract, the genital organs and the like, but are normally eliminated by the host immune system. However, in a state of increased susceptibility to infection, proliferation of mucosal epithelial cells is induced by virus proteins E6 and E7, so that a papilloma is formed. This may lead to recurrent respiratory papillomatosis or condyloma acuminatum. On rare occasions, a malignant change may occur (Non Patent Literature 3).

HPV infection is initiated as the L1 protein, forming a capsid, adsorbs to heparan sulfate proteoglycan, present on the surfaces of host cell membranes (Non Patent Literature 10). Neutralizing antibodies, engaged in protection against HIV infection by targeting the L1 protein, are currently marketed as prophylactic vaccines that contain a virus-like particle (VLP) antigen of the L1 protein as a medicinal ingredient. The currently available prophylactic vaccines also contain L1 VLP antigens of genotypes 6 and 11, so that the frequency of surgical operations in treatment of recurrent respiratory papillomatosis is reduced (Non Patent Literature 11).

The host protective immunity against HPV infection consists of induction of a neutralizing antibody and cytotoxic T cells (CTLs) or helper T cells. In particular, nonstructural proteins E6 and E7 are target antigens in CTL induction, and attract attention as therapeutic vaccine antigens for uterine cervical cancer and cervical dysplasia caused by HPV infection (Non Patent Literature 12). Also, these nonstructural proteins attract attention as therapeutic vaccine antigens for the treatment of recurrent respiratory papillomatosis (Non Patent Literature 13).

Patent Literature 1 discloses an E6 and E7 fusion antigen gene sequence of HPV genotypes 6, 11, 16, 18, 31, 33, 39, 45, 52 and 58. In the gene sequence disclosed in the literature, an IgE leader sequence is added to the N-terminus of E6, and a furin peptidase cleavage site is inserted between the translated region sequences of E6 and E7. A mutation is inserted between the p53 binding region of E6 and the pRb binding region of E7 to eliminate the canceration activity of E6 and E7. The gene sequence is introduced into a mammalian expression plasmid to form a DNA gene vaccine against HPV, and its drug effect in a mouse model is evaluated. The immunization is performed by intramuscular administration into the mouse's femoral area by electroporation.

### Citation List

### Non Patent Literature

Non Patent Literature 1: Virology 2013; 445: 2e10.
Non Patent Literature 2: J Natl Cancer Inst 1995; 87: 796-802.

Non Patent Literature 3: Virus Res. 2017, 231: 119-127.
Non Patent Literature 4: J Clin Virol 2005; 32 (Suppl.1): S7e15.
Non Patent Literature 5: Cell 1990; 63: 1129e36.
Non Patent Literature 6: Cancer Res 1996; 56: 4620e4.
Non Patent Literature 7: J Virol, 1989, p.2650-2656
Non Patent Literature 8: J Virol, 1992, p.1329-1335
Non Patent Literature 9: J Virol, 1994, p.5698-5705
Non Patent Literature 10: Proc Natl Acad Sci U. S. A 2009; 106: 20458e63.
Non Patent Literature 11: J Infect Dis. 2019, 219(7): 1016-1025.
Non Patent Literature 12: Nat Rev Cancer. 2006, 6(10): 753-763.
Non Patent Literature 13: Hum Vaccin Immunother. 2012, 8(4): 470-478.

Patent Literature 1: JP 2016-512553

Summary of Invention

Technical Problem

An object of the present invention is to provide a vaccine for preventing and/or treating infection with human papillomavirus type 6 and/or type 11.

Solution to Problem

The present inventors have found that by administrating lipid particles encapsulating mRNA encoding E6-E7 antigens of human papillomavirus type 6 and/or type 11 to mice, an immune response specific to the antigens is induced, leading to completion of the present invention.
The gist of the present invention is as follows.

(1) A lipid particle encapsulating a nucleic acid capable of expressing an E6 antigen and an E7 antigen of human papillomavirus, wherein a lipid comprises a cationic lipid having the general formula (Ia), or a pharmaceutically acceptable salt thereof:

[Formula 1]

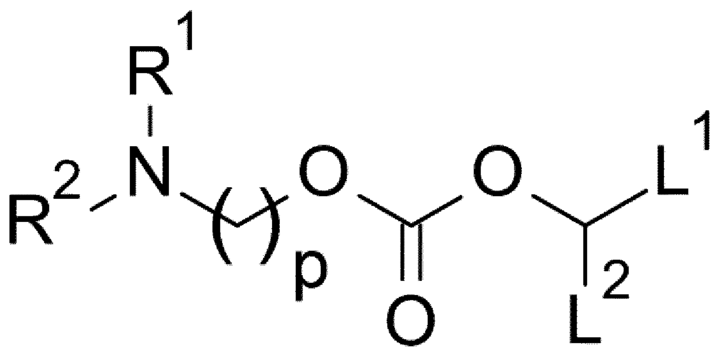

(Ia)

wherein

$R^1$ and $R^2$ each independently represent a C1-C3 alkyl group;
$L^1$ represents a C17-C19 alkenyl group, optionally having one or more C2-C4 alkanoyloxy groups;
$L^2$ represents a C10-C19 alkyl group, optionally having one or more C2-C4 alkanoyloxy groups, or a C10-C19 alkenyl group, optionally having one or more C2-C4 alkanoyloxy groups; and
p is 3 or 4.

(2) The particle according to (1), wherein each of $R^1$ and $R^2$ in the general formula (Ia) is a methyl group.
(3) The particle according to (1) or (2), wherein p in the general formula (Ia) is 3.

(4) The particle according to any one of (1) to (3), wherein $L^1$ in the general formula (Ia) is a C17-C19 alkenyl group, optionally having one or more acetoxy groups.

(5) The particle according to any one of (1) to (4), wherein $L^2$ in the general formula (Ia) is a C10-C12 alkyl group, optionally having one or more acetoxy groups, or a C10-C19 alkenyl group, optionally having one or more acetoxy groups.

(6) The particle according to any one of (1) to (4), wherein $L^2$ in the general formula (Ia) is a C10-C12 alkyl group optionally having one or more acetoxy groups, or a C17-C19 alkenyl group, optionally having one or more acetoxy groups.

(7) The particle according to any one of (1) to (6), wherein $L^1$ in the general formula (Ia) is a (R)-11-acetyloxy-cis-8-heptadecenyl group, a cis-8-heptadecenyl group, or a (8Z,11Z)-heptadecadienyl group.

(8) The particle according to any one of (1) to (7), wherein $L^2$ in the general formula (Ia) is a decyl group, a cis-7-decenyl group, a dodecyl group, or a (R)-11-acetyloxy-cis-8-heptadecenyl group.

(9) The particle according to (1), wherein the cationic lipid has the following structural formula:

[Formula 2]

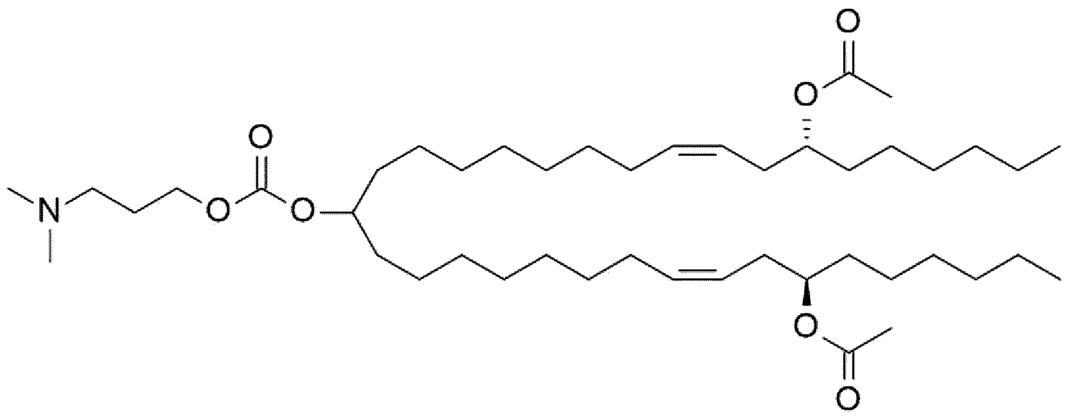

(10) The particle according to (1), wherein the cationic lipid has the following structural formula:

[Formula 3]

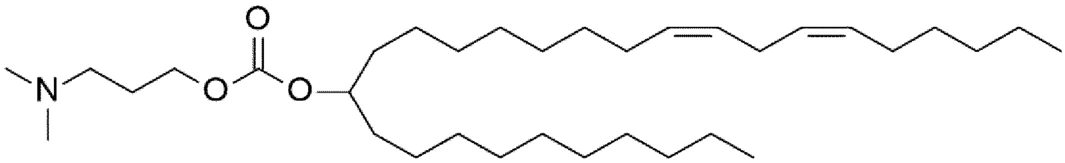

(11) The particle according to (1), wherein the cationic lipid has the following structural formula:

[Formula 4]

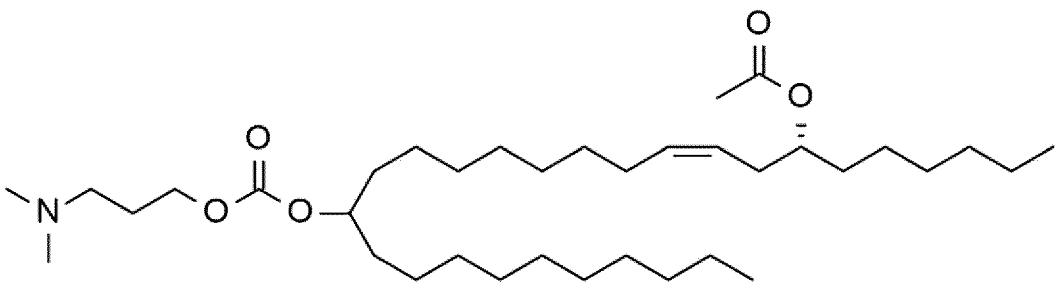

(12) The particle according to (9) or (10), wherein the lipid further comprises an amphipathic lipid, a sterol and a

PEG lipid.

(13) The particle according to (11), wherein the lipid further comprises an amphipathic lipid, a sterol and a PEG lipid.

(14) The particle according to (12), wherein the amphipathic lipid is at least one selected from the group consisting of distearoyl phosphatidylcholine, dioleoyl phosphatidylcholine and dioleoyl phosphatidylethanolamine.

(15) The particle according to (13), wherein the amphipathic lipid is at least one selected from the group consisting of distearoyl phosphatidylcholine, dioleoyl phosphatidylcholine and dioleoyl phosphatidylethanolamine.

(16) The particle according to (12) or (14), wherein the sterol is cholesterol.

(17) The particle according to (13) or (15), wherein the sterol is cholesterol.

(18) The particle according to any one of (12), (14) and (16), wherein the PEG lipid is 1,2-dimyristoyl-sn-glycelol methoxypolyethylene glycol and/or N-[methoxypoly(ethylene glycol) 2000]carbamoyl]-1,2-dimyristyloxypropyl-3-amine.

(19) The particle according to any one of (13), (15) and (17), wherein the PEG lipid is 1,2-dimyristoyl-sn-glycelol methoxypolyethylene glycol and/or N-[methoxypoly(ethylene glycol) 2000]carbamoyl]-1,2-dimyristyloxypropyl-3-amine.

(20) The particle according to any one of (12) to (19), wherein the lipid composition of the amphipathic lipid, the sterol, the cationic lipid and the PEG lipid is amphipathic lipid: 5 to 25%, sterol: 10 to 55%, cationic lipid: 40 to 65% and PEG lipid: 1 to 5% on a molar amount basis.

(21) The particle according to (20), wherein the proportion of the amphipathic lipid is 10 to 25%.

(22) The particle according to any one of (12), (14), (16) and (18), wherein the lipid composition of the amphipathic lipid, the sterol, the cationic lipid and the PEG lipid is amphipathic lipid: 5 to 15%, sterol: 35 to 50%, cationic lipid: 40 to 55% and PEG lipid: 1 to 3% on a molar amount basis.

(23) The particle according to (22), wherein the proportions of the amphipathic lipid, the sterol, the cationic lipid and the PEG lipid are 10 to 15%, 35 to 45%, 40 to 50% and 1 to 2.5%, respectively.

(24) The particle according to (23), wherein the proportion of the PEG lipid is 1 to 2%.

(25) The particle according to any one of (13), (15), (17) and (19), wherein the lipid composition of the amphipathic lipid, the sterol, the cationic lipid and the PEG lipid is amphipathic lipid: 10 to 25%, sterol: 10 to 50%, cationic lipid: 40 to 65% and PEG lipid: 1 to 3% on a molar amount basis.

(26) The particle according to (25), wherein the proportions of the sterol, the cationic lipid and the PEG lipid are 10 to 45%, 42.5 to 65% and 1 to 2.5%, respectively.

(27) The particle according to (26), wherein the proportion of the PEG lipid is 1 to 2%.

(28) The particle according to any one of (20) to (27), wherein the ratio of the total weight of lipids to the weight of the nucleic acid is from 15 to 30.

(29) The particle according to (28), wherein the ratio of the total weight of lipids to the weight of the nucleic acid is from 15 to 25.

(30) The particle according to (29), wherein the ratio of the total weight of lipids to the weight of the nucleic acid is from 17.5 to 22.5.

(31) The particle according to any one of (1) to (30), wherein the human papillomavirus is HPV type 6.

(32) The particle according to (31), wherein the human papillomavirus is HPV type 6, and the E6 antigen of HPV type 6 consists of an amino acid sequence having an identity of at least 95% with the amino acid sequence of SEQ ID NO: 12.

(33) The particle according to (31) or (32), wherein the human papillomavirus is HPV type 6, and the E7 antigen of HPV type 6 consists of an amino acid sequence having an identity of at least 95% with the amino acid sequence of SEQ ID NO: 13.

(34) The particle according to any one of (31) to (33), wherein the human papillomavirus is HPV type 6, and the nucleic acid capable of expressing the E6 antigen and the E7 antigen of the human papillomavirus encodes a fusion protein of the E6 antigen and the E7 antigen of HPV type 6, which consists of an amino acid sequence having an identity of at least 95% with the amino acid sequence of SEQ ID NO: 17.

(35) The particle according to any one of (31) to (34), wherein the human papillomavirus is HPV type 6, and the nucleic acid capable of expressing the E6 antigen and the E7 antigen of HPV type 6 is an mRNA comprising a cap structure (Cap), a 5' untranslated region (5'-UTR), a leader sequence, a translated region of E6, a protease cleavage sequence (furin cleavage site), a translated region of E7, a 3' untranslated region (3'-UTR) and a poly A tail (polyA).

(36) The particle according to (35), wherein the sequence of the nucleic acid capable of expressing the E6 antigen and the E7 antigen of HPV type 6 consists of a nucleotide sequence having an identity of at least 90% with the sequence of SEQ ID NO: 5.

(37) The particle according to any one of (31) to (34), wherein the human papillomavirus is HPV type 6, and the nucleic acid capable of expressing the E6 antigen and the E7 antigen of HPV type 6 is an mRNA having a structure comprising a cap structure (Cap), a 5' untranslated region (5'-UTR), a leader sequence, a translated region of E6, a protease cleavage sequence (furin cleavage site), a translated region of E7 and a 3' untranslated region (3'-UTR).

(38) The particle according to (37), wherein the structure comprising a cap structure (Cap), a 5' untranslated region (5'-UTR), a leader sequence, a translated region of E6, a protease cleavage sequence (furin cleavage site), a translated region of E7 and a 3' untranslated region (3'-UTR) consists of a nucleotide sequence having an identity of at least 90% with the sequence of residues 1 to 1018 in SEQ ID NO: 5.
(39) The particle according to any one of (1) to (30), wherein the human papillomavirus is HPV type 11.
(40) The particle according to (39), wherein the human papillomavirus is HPV type 11, and the E6 antigen of HPV type 11 consists of an amino acid sequence having an identity of at least 95% with the amino acid sequence of SEQ ID NO: 14.
(41) The particle according to (39) or (40), wherein the human papillomavirus is HPV type 11, and the E7 antigen of HPV type 11 consists of an amino acid sequence having an identity of at least 95% with the amino acid sequence of SEQ ID NO: 15.
(42) The particle according to any one of (39) to (41), wherein the human papillomavirus is HPV type 11, and the nucleic acid capable of expressing the E6 antigen and the E7 antigen of the human papillomavirus encodes a fusion protein of the E6 antigen and the E7 antigen of HPV type 11, which consists of an amino acid sequence having an identity of at least 95% with the amino acid sequence of SEQ ID NO: 18.
(43) The particle according to any one of (39) to (42), wherein the human papillomavirus is HPV type 11, and the nucleic acid capable of expressing the E6 antigen and the E7 antigen of HPV type 11 is an mRNA comprising a cap structure (Cap), a 5' untranslated region (5'-UTR), a leader sequence, a translated region of E6, a protease cleavage sequence (furin cleavage site), a translated region of E7, a 3' untranslated region (3'-UTR) and a poly A tail (polyA).
(44) The particle according to (43), wherein the sequence of the nucleic acid capable of expressing the E6 antigen and the E7 antigen of HPV type 11 consists of a nucleotide sequence having an identity of at least 90% with the sequence of SEQ ID NO: 8.
(45) The particle according to any one of (39) to (42), wherein the human papillomavirus is HPV type 11, and the nucleic acid capable of expressing the E6 antigen and the E7 antigen of HPV type 11 is an mRNA having a structure comprising a cap structure (Cap), a 5' untranslated region (5'-UTR), a leader sequence, a translated region of E6, a protease cleavage sequence (furin cleavage site), a translated region of E7 and a 3' untranslated region (3'-UTR).
(46) The particle according to (45), wherein the structure comprising a cap structure (Cap), a 5' untranslated region (5'-UTR), a leader sequence, a translated region of E6, a protease cleavage sequence (furin cleavage site), a translated region of E7 and a 3' untranslated region (3'-UTR) consists of a nucleotide sequence having an identity of at least 90% with the sequence of residues 1 to 1018 in SEQ ID NO: 8.
(47) The particle according to any one of (1) to (30), wherein the human papillomavirus is HPV type 6 and type 11.
(48) The particle according to (47), wherein the human papillomavirus is HPV type 6 and type 11, and the nucleic acid capable of expressing the E6 antigen and the E7 antigen of the human papillomavirus encodes a fusion protein of the E6 antigen and the E7 antigen of HPV type 6 and the E6 antigen and the E7 antigen of HPV type 11, which consists of an amino acid sequence having an identity of at least 95% with the amino acid sequence of SEQ ID NO: 19.
(49) The particle according to (47) or (48), wherein the human papillomavirus is HPV type 6 and type 11, and the nucleic acid capable of expressing the E6 antigen and the E7 antigen of HPV type 6 and the E6 antigen and the E7 antigen of HPV type 11 is an mRNA comprising a cap structure (Cap), a 5' untranslated region (5'-UTR), a leader sequence, a translated region of E6 of HPV type 6, a translated region of E7 of HPV type 6, a protease cleavage sequence (furin cleavage site), a translated region of E6 of HPV type 11, a translated region of E7 of HPV type 11, a 3' untranslated region (3'-UTR) and a poly A tail (polyA).
(50) The particle according to (49), wherein the sequence of the nucleic acid capable of expressing the E6 antigen and the E7 antigen of HPV type 6 and the E6 antigen and the E7 antigen of HPV type 11 consists of a nucleotide sequence having an identity of at least 90% with the sequence of SEQ ID NO: 11.
(51) The particle according to (47) or (48), wherein the human papillomavirus is HPV type 6 and type 11, and the nucleic acid capable of expressing the E6 antigen and the E7 antigen of HPV type 6 and the E6 antigen and the E7 antigen of HPV type 11 is an mRNA having a structure comprising a cap structure (Cap), a 5' untranslated region (5'-UTR), a leader sequence, a translated region of E6 of HPV type 6, a translated region of E7 of HPV type 6, a protease cleavage sequence (furin cleavage site), a translated region of E6 of HPV type 11, a translated region of E7 of HPV type 11 and a 3' untranslated region (3'-UTR).
(52) The particle according to (51), wherein the structure comprising a cap structure (Cap), a 5' untranslated region (5'-UTR), a leader sequence, a translated region of E6 of HPV type 6, a translated region of E7 of HPV type 6, a protease cleavage sequence (furin cleavage site), a translated region of E6 of HPV type 11, a translated region of E7 of HPV type 11 and a 3' untranslated region (3'-UTR) consists of a nucleotide sequence having an identity of at least 90% with the sequence of residues 1 to 1798 of the sequence of SEQ ID NO: 11.
(53) The particle according to any one of (1) to (52), wherein the nucleic acid comprises at least one modified nucleotide.
(54) The particle according to (53), wherein the modified nucleotide comprises at least one of pyrimidine nucleotide

substituted at the 5-position and/or pseudouridine optionally substituted at the 1-position.

(55) The particle according to (53), wherein the modified nucleotide comprises at least one selected from the group consisting of 5-methylcytidine, 5-methoxyuridine, 5-methyluridine, pseudouridine and 1-alkylpseudouridine.

(56) The particle according to (53), wherein the modified nucleotide comprises at least one selected from the group consisting of 5-methylcytidine, 5-methyluridine and 1-methylpseudouridine.

(57) The particle according to any one of (1) to (56), which has an average particle size of 30 to 300 nm.

(58) Use of the particle according to any one of (1) to (57), for producing a composition for preventing and/or treating infection with human papillomavirus.

(59) Use of the particle according to any one of (1) to (57), for producing a composition for preventing and/or treating a disease caused by infection with human papillomavirus.

(60) The use of the particle according to (59), wherein the disease caused by infection with human papillomavirus is recurrent respiratory papillomatosis or condyloma acuminatum.

(61) The use of the particle according to any one of (58) to (60), wherein the infection is caused by human papillomavirus of type 6 or type 11.

(62) A composition comprising the particle according to any one of (1) to (57).

(63) The composition according to (62) for expressing the E6 antigen and the E7 antigen of human papillomavirus in vivo or in *vitro.*

(64) The composition according to (62) or (63) for use as a medicament.

(65) The composition according to (64) for inducing an immune reaction against human papillomavirus.

(66) The composition according to (64) or (65) for preventing and/or treating infection with human papillomavirus.

(67) The composition according to (64) or (65) for preventing and/or treating a disease caused by infection with human papillomavirus.

(68) The composition according to (67), wherein the disease caused by infection with human papillomavirus is recurrent respiratory papillomatosis or condyloma acuminatum.

(69) The composition according to any one of (66) to (68), wherein the infection is caused by human papillomavirus of type 6 or type 11.

(70) A method for expressing an E6 antigen and an E7 antigen of human papillomavirus *in vitro,* comprising introducing the composition according to (62) or (63) into cells.

(71) A method for expressing an E6 antigen and an E7 antigen of human papillomavirus *in vivo,* comprising administering the composition according to any one of (62) to (66) to a mammal.

(72) A method for inducing an immune reaction against human papillomavirus, comprising administering the composition according to (64) or (65) to a mammal.

(73) A method for preventing and/or treating an infection with human papillomavirus, comprising administering the composition according to any one of (64) to (69) to a mammal.

## Advantageous Effects of Invention

By the present invention, infection with human papillomavirus type 6 and/or type 11 can be prevented and/or treated. In addition, by the present invention, diseases caused by infection with human papillomavirus type 6 and/or type 11 (recurrent respiratory papillomatosis, condyloma acuminatum and the like) can be prevented and/or treated. The particles of the present invention have excellent properties in terms of metabolic stability, *in vitro* activity, *in vivo* activity, rapidity of onset of a drug effect, persistence of a drug effect, physical stability, drug interaction, safety and the like, and are useful as a medicament for preventing and/or treating the above-described diseases.

## Brief Description of Drawings

[Figure 1] Figure 1 shows the HPV6E6- and HPV11E6-specific INF-γ production levels of mRNA-encapsulating nucleic acid lipid particles in C57BL/6 mice. Examples 4, 7, 10: group receiving mRNA-encapsulating nucleic acid lipid particles, NC: negative control group receiving a buffer, No peptides: peptide-non-treated negative control group, HPV6E6 peptides: HPV6E6 pool peptide-treated group, HPV11E6 peptides: HPV11E6 pool peptide-treated group. The bar graph represents the average value for each group, and the error bar represents the standard deviation.

[Figure 2] Figure 2 shows the HPV6E6- and HPV11E6-specific INF-γ production levels of mRNA-encapsulating nucleic acid lipid particles in C57BL/6 mice. Examples 5, 8, 11: group receiving mRNA-encapsulating nucleic acid lipid particles, NC: negative control group receiving a buffer, No peptides: peptide-non-treated negative control group, HPV6E6 peptides: HPV6E6 pool peptide-treated group, HPV11E6 peptides: HPV11E6 pool peptide-treated

group. The bar graph represents the average value for each group, and the error bar represents the standard deviation.

[Figure 3] Figure 3 shows the HPV6E6- and HPV11E6-specific INF-γ production levels of mRNA-encapsulating nucleic acid lipid particles in C57BL/6 mice. Examples 6, 9, 12: group receiving mRNA-encapsulating nucleic acid lipid particles, NC: negative control group receiving a buffer, No peptides: peptide-non-treated negative control group, HPV6E6 peptides: HPV6E6 pool peptide-treated group, HPV11E6 peptides: HPV11E6 pool peptide-treated group. The bar graph represents the average value for each group, and the error bar represents the standard deviation.

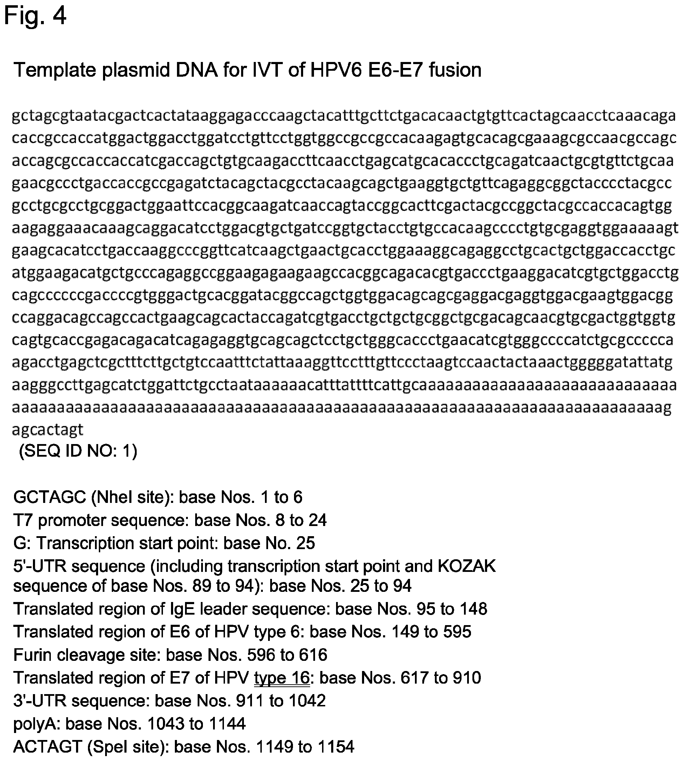

[Figure 4] Figure 4 shows a nucleotide sequence of template plasmid DNA for IVT of HPV6 E6-E7 fusion (SEQ ID NO: 1).

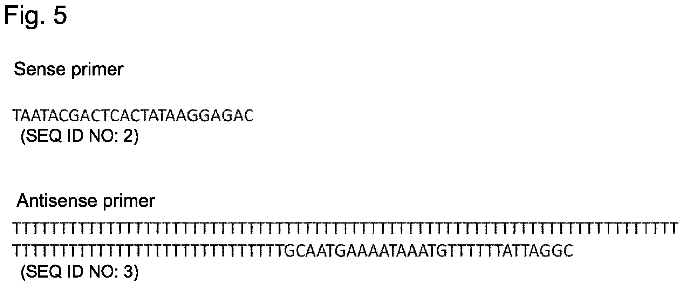

[Figure 5] Figure 5 shows nucleotide sequences of a sense primer (SEQ ID NO: 2) and an antisense primer (SEQ ID NO: 3) .

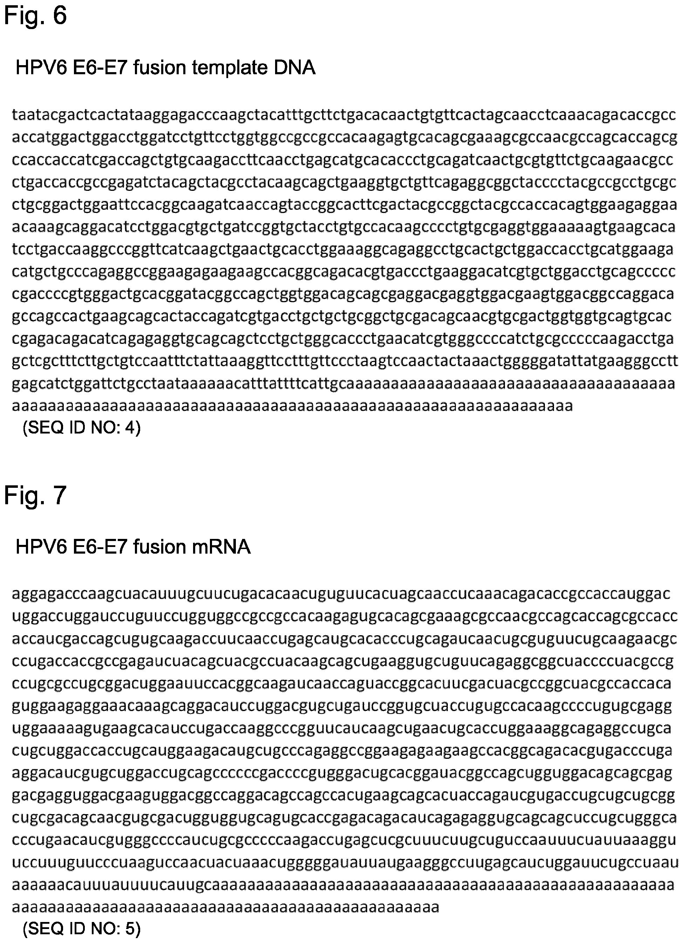

[Figure 6] Figure 6 shows the nucleotide sequence of HPV6 E6-E7 fusion template DNA (SEQ ID NO: 4).

[Figure 7] Figure 7 shows the nucleotide sequence of HPV6 E6-E7 fusion mRNA (SEQ ID NO: 5).

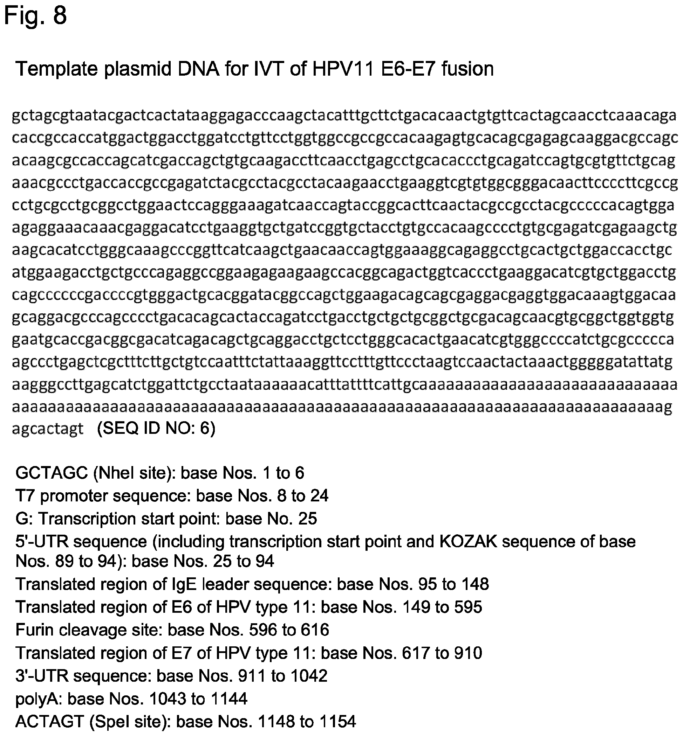

[Figure 8] Figure 8 shows the nucleotide sequence of template plasmid DNA for IVT of HPV6 E6-E7 fusion (SEQ ID NO: 6).

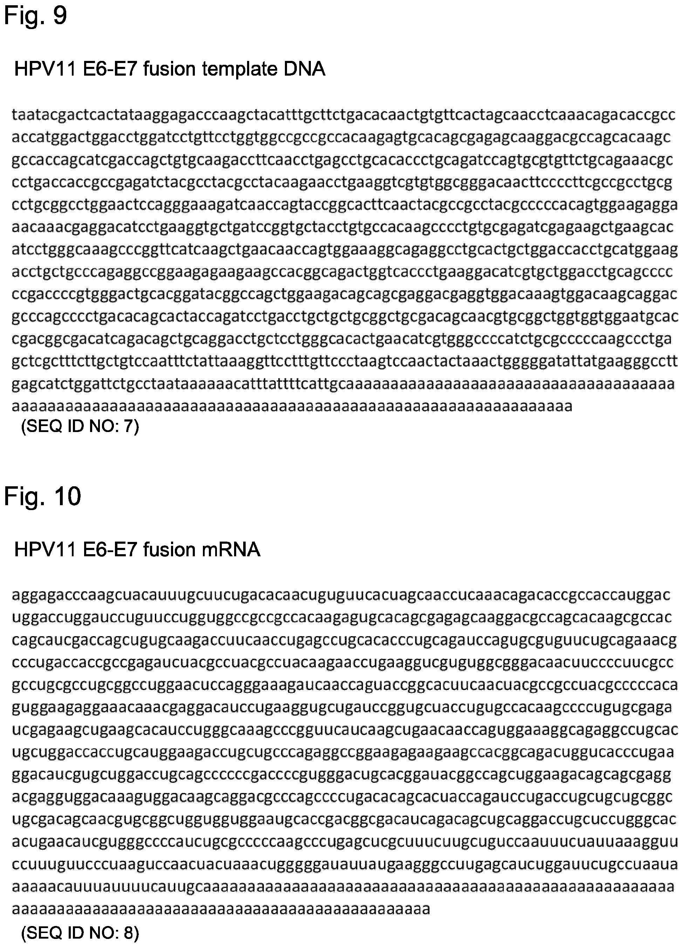

[Figure 9] Figure 9 shows the nucleotide sequence of HPV11 E6-E7 fusion template DNA (SEQ ID NO: 7).

[Figure 10] Figure 10 shows the nucleotide sequence of HPV11 E6-E7 fusion mRNA (SEQ ID NO: 8).

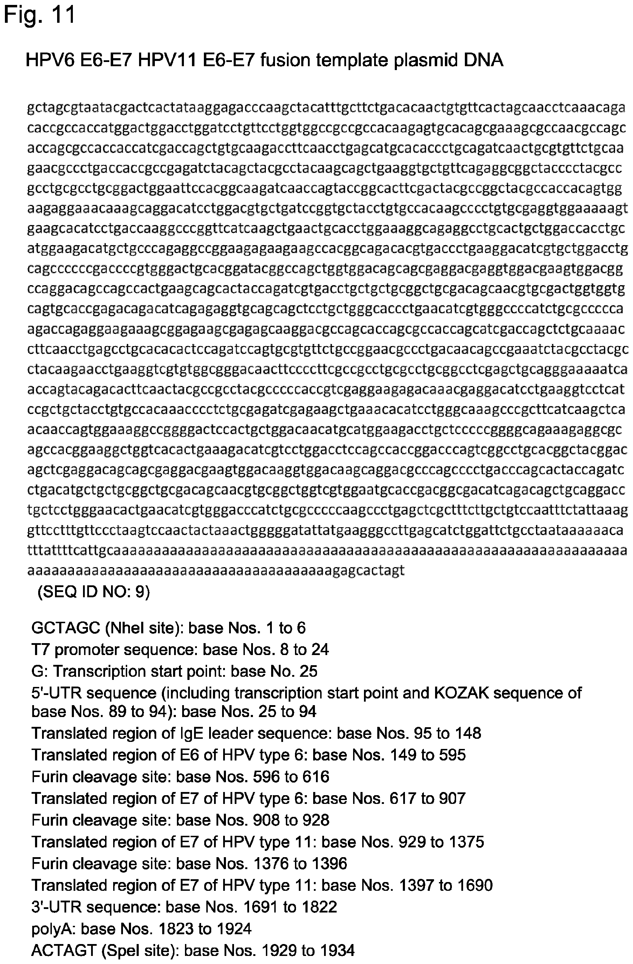

[Figure 11] Figure 11 shows the nucleotide sequence of HPV6 E6-E7 HPV11 E6-E7 fusion template plasmid DNA (SEQ ID NO: 9).

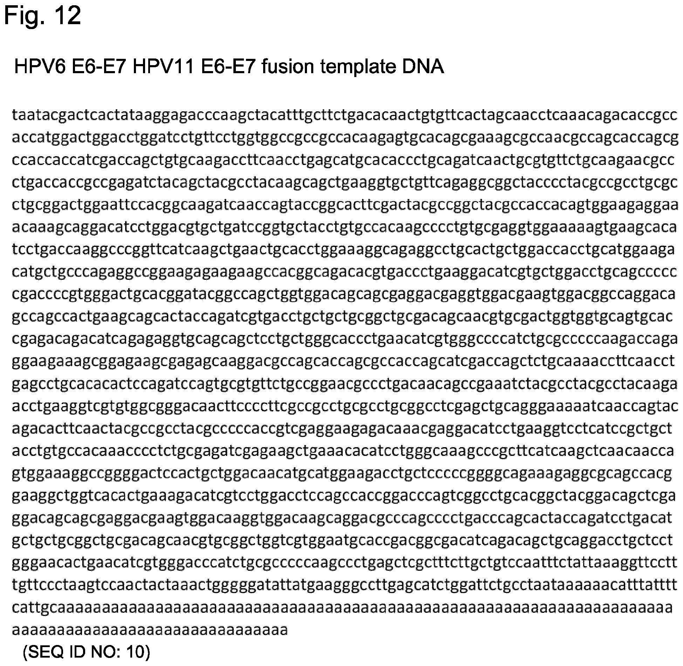

[Figure 12] Figure 12 shows the nucleotide sequence of HPV6 E6-E7 HPV11 E6-E7 fusion template DNA (SEQ ID NO: 10) .

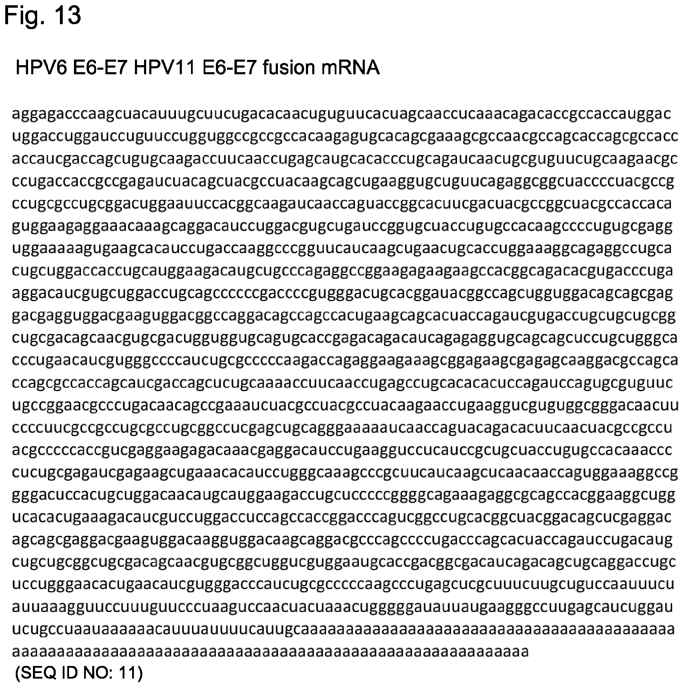

[Figure 13] Figure 13 shows the nucleotide sequence of HPV6 E6-E7 HPV11 E6-E7 fusion mRNA (SEQ ID NO: 11).

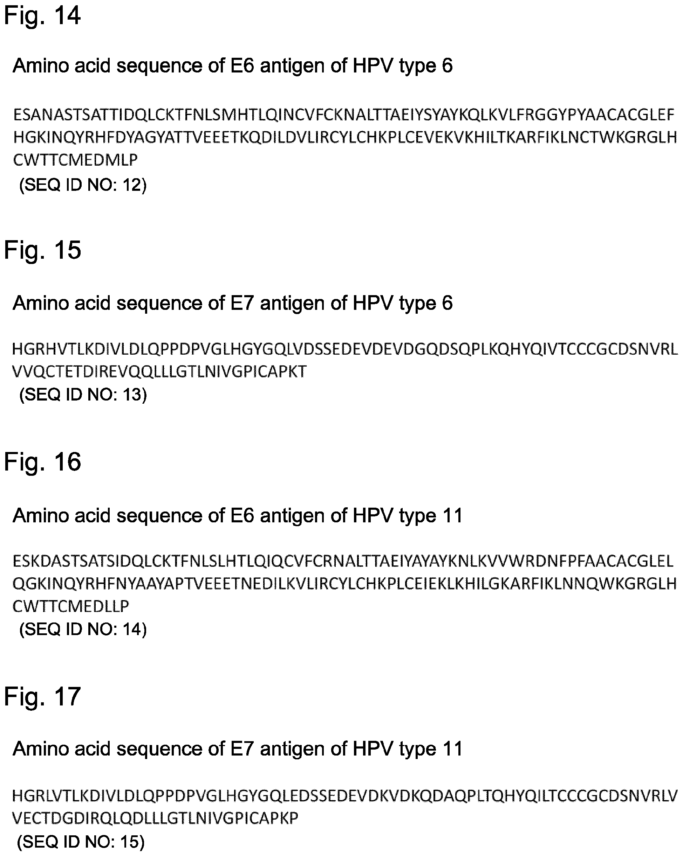

[Figure 14] Figure 14 shows the amino acid sequence of an E6 antigen of HPV type 6 (SEQ ID NO: 12).

[Figure 15] Figure 15 shows the amino acid sequence of an E7 antigen of HPV type 6 (SEQ ID NO: 13).

[Figure 16] Figure 16 shows the amino acid sequence of the E6 antigen of HPV type 11 (SEQ ID NO: 14).

[Figure 17] Figure 17 shows the amino acid sequence of the E7 antigen of HPV type 11 (SEQ ID NO: 15).

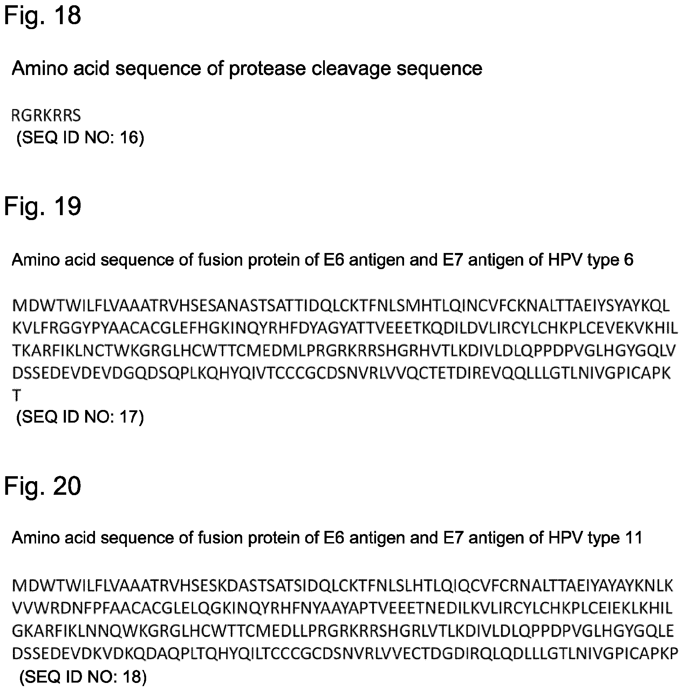

[Figure 18] Figure 18 shows the amino acid sequence of a protease cleavage sequence (SEQ ID NO: 16).

[Figure 19] Figure 19 shows the amino acid sequence of a fusion protein of the E6 antigen and the E7 antigen of HPV type 6 (SEQ ID NO: 17).

[Figure 20] Figure 20 shows the amino acid sequence of a fusion protein of the E6 antigen and the E7 antigen of HPV type 11 (SEQ ID NO: 18).

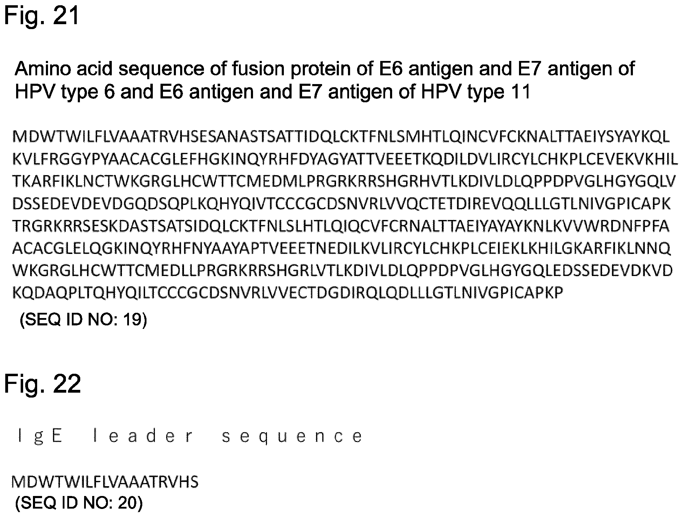

[Figure 21] Figure 21 shows the amino acid sequence of a fusion protein of the E6 antigen and the E7 antigen of HPV type 6 and the E6 antigen and the E7 antigen of HPV type 11 (SEQ ID NO: 19).

[Figure 22] Figure 22 shows the IgE leader sequence (SEQ ID NO: 20).

Description of Embodiments

Hereinafter, the present invention will be described in detail.

The terms used herein are described below.

The term "lipid particle" as used herein denotes a particle comprising an amphipathic lipid, a sterol, a cationic lipid and a PEG lipid as a constituent lipid.

The term "capable of expressing" as used herein means that a target protein can be produced in cells *in vitro* or *in vivo.*

The term "C1-C3 alkyl group" denotes a linear or branched alkyl group having 1 to 3 carbon atoms. Examples thereof include a methyl group, an ethyl group, a propyl group, and an isopropyl group.

The term "C2-C4 alkanoyl group" denotes an alkanoyl group having 2 to 4 carbon atoms. Examples thereof include an acetyl group, a propionyl group, a butyryl group, and an isobutyryl group.

The term "C2-C4 alkanoyloxy group" denotes a group in which the C2-C4 alkanoyl group is bonded to an oxygen atom. Examples thereof include an acetyloxy group, a propionyloxy group, a butyryloxy group, and an isobutyryloxy group.

The term "C17-C19 alkenyl group" denotes a linear or branched alkenyl group having 17 to 19 carbon atoms. The C17-C19 alkenyl group herein includes all of a C17-C19 alkadienyl group, a C17-C19 alkatrienyl group and a C17-C19 alkatetraenyl group. Examples thereof include a heptadecenyl group, an octadecenyl group, a nonadecenyl group, a heptadecadienyl group, an octadecadienyl group, a nonadecadienyl group, a heptadecatrienyl group, an octadecatrienyl group, and a nonadecatrienyl group.

The term "C17-C19 alkenyl group optionally having one or more C2-C4 alkanoyloxy groups" denotes a group in which a hydrogen atom at any position on the C17-C19 alkenyl group is replaced with the C2-C4 alkanoyloxy group.

Examples thereof include a 11-acetyloxy-8-heptadecenyl group, and a 11-propionyloxy-8-heptadecenyl group.

The term "C10-C19 alkyl group" denotes a linear or branched alkyl group having 10 to 19 carbon atoms. Examples thereof include a decyl group, an undecyl group, a dodecyl group, a tridecyl group, a tetradecyl group, a pentadecyl group, a hexadecyl group, a heptadecyl group, an octadecyl group, a nonadecyl group.

The term "C10-C19 alkyl group optionally having one or more C2-C4 alkanoyloxy groups" denotes a group in which a hydrogen atom at any position on the C10-C19 alkyl group is replaced with the C2-C4 alkanoyloxy group.

The term "C10-C19 alkenyl group" denotes a linear or branched alkenyl group having 10 to 19 carbon atoms. The C10-C19 alkenyl group herein includes all of a C10-C19 alkadienyl group, a C10-C19 alkatrienyl group and a C10-C19 alkatetraenyl group. Examples thereof include a decenyl group, an undecenyl group, a dodecenyl group, a tridecenyl group, a tetradecenyl group, a pentadecenyl group, a hexadecenyl group, a heptadecenyl group, an octadecenyl group, a nonadecenyl group, a decadienyl group, an undecadienyl group, a dodecadienyl group, a tridecadienyl group, a tetradecadienyl group, a pentadecadienyl group, a hexadecadienyl group, a heptadecadienyl group, an octadecadienyl group, a nonadecadienyl group, a decatrienyl group, an undecatrienyl group, a dodecatrienyl group, a tridecatrienyl group, a tetradecatrienyl group, a pentadecatrienyl group, a hexadecatrienyl group, a heptadecatrienyl group, an octadecatrienyl group, and a nonadecatrienyl group.

The term "C10-C19 alkenyl group optionally having one or more C2-C4 alkanoyloxy groups" denotes a group in which a hydrogen atom at any position on the C10-C19 alkenyl group is replaced with the C2-C4 alkanoyloxy group. Examples thereof include a 11-acetyloxy-8-heptadecenyl group, and a 11-propionyloxy-8-heptadecenyl group.

The term "C17-C19 alkenyl group optionally having one or more acetyloxy groups" denotes a group in which a hydrogen atom at any position on the C17-C19 alkenyl group is replaced with an acetyloxy group. Examples thereof include a 11-acetyloxy-8-heptadecenyl group, and a 11-propionyloxy-8-heptadecenyl group.

The term "C10-C12 alkyl group optionally having one or more acetyloxy groups" denotes a group in which a hydrogen atom at any position on the C10-C12 alkyl group is replaced with an acetyloxy group.

The term "C10-C19 alkenyl group optionally having one or more acetyloxy groups" denotes a group in which a hydrogen atom at any position on the C10-C19 alkenyl group is replaced with an acetyloxy group. Examples thereof include a 11-acetyloxy-8-heptadecenyl group, and a 11-propionyloxy-8-heptadecenyl group.

The term "treatment" as used herein means that in a patient having developed an infection with a virus, bacteria or the like, or a disease caused by the infection (for example, precancerous lesion or cancer), the clinical symptoms of such a disease are cured, caused to remit, alleviated and/or delayed from worsening.

The term "prevention" as used herein means reducing incidence of a disease caused by an infection with a virus, bacteria or the like. The prevention includes lowering the risk of progression of a disease caused by an infection with a virus, bacteria or the like, or reducing aggravation of such a disease. The particles of the present invention are effective in prevention and/or treatment of the disease because they induce a preventive immune reaction.

The term "identity" as used herein refers to a relationship between two or more nucleotide sequences or amino acid sequences which is determined by comparison between the sequences as known in the art. In the art, the identity also means, as the case may be, a degree of sequence relatedness between nucleic acid molecules or polypeptides when determined by a match between two or more nucleotide sequences or two or more amino acid sequences in one row. The identity can be evaluated by calculating a percentage of the exact match between the smallest of two or more sequences and a gap alignment (if present) addressed by a specific mathematical model or computer program (i.e., "algorithm"). Specifically, software such as Clustal W2 provided by European Molecular Biology Laboratory-European Bioinformatics Institute (EMBL-EBI) may be used to evaluate the identity, but the tools for calculation of identity are not limited thereto as long as they are used by those skilled in the art.

The present invention provides lipid particles encapsulating a nucleic acid capable of expressing an E6 antigen and an E7 antigen of human papillomavirus type 6 and/or type 11, wherein a lipid comprises a cationic lipid having the general formula (Ia), or a pharmaceutically acceptable salt thereof.

[Formula 5]

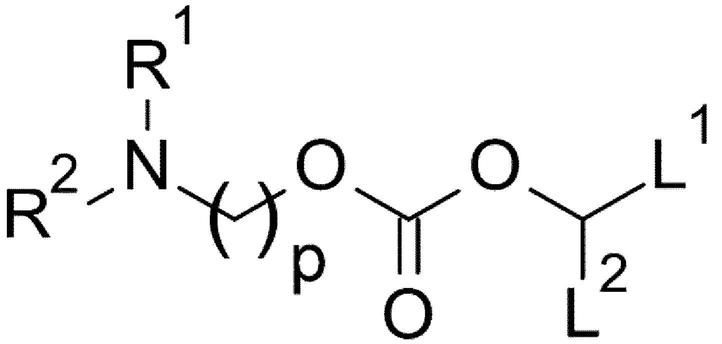

(Ia)

wherein

- $R^1$ and $R^2$ each independently represent a C1-C3 alkyl group;
- $L^1$ represents a C17-C19 alkenyl group, optionally having one or more C2-C4 alkanoyloxy groups;
- $L^2$ represents a C10-C19 alkyl group optionally having one or more C2-C4 alkanoyloxy groups, or a C10-C19 alkenyl group, optionally having one or more C2-C4 alkanoyloxy groups; and
- p is 3 or 4.

In the general formula (Ia), $R^1$ and $R^2$ each independently represent a C1-C3 alkyl group, preferably a methyl group.

In the general formula (Ia), p is 3 or 4, preferably 3.

In the general formula (Ia), $L^1$ represents a C17-C19 alkenyl group, optionally having one or more C2-C4 alkanoyloxy groups, preferably a C17-C19 alkenyl group, optionally having one or more acetoxy groups. Specifically, a (R)-11-acetyloxy-cis-8-heptadecenyl group, a cis-8-heptadecenyl group, a (8Z,11Z)-heptadecadienyl group or the like can be exemplified as $L^1$.

In the general formula (Ia), $L^2$ represents a C10-C19 alkyl group, optionally having one or more C2-C4 alkanoyloxy groups, or a C10-C19 alkenyl group, optionally having one or more C2-C4 alkanoyloxy groups, preferably a C10-C12 alkyl group, optionally having one or more acetoxy groups, or a C10-C19 alkenyl group, optionally having one or more acetoxy groups. It is also preferable that $L^2$ in the general formula (Ia) be a C10-C12 alkyl group, optionally having one or more acetoxy groups, or a C17-C19 alkenyl group optionally having one or more acetoxy groups. Specifically, a decyl group, a cis-7-decenyl group, a dodecyl group, a (R)-11-acetyloxy-cis-8-heptadecenyl group or the like can be exemplified as $L^2$.

Specifically, (7R,9Z,26Z,29R)-18-({[3-(dimethylamino)propoxy]carbonyl}oxy)pentatriaconta-9,26-diene-7,29-diyl diacetate, 3-dimethylaminopropyl(9Z,12Z)-octaoctacosa-19,22-dien-11-yl carbonate, and (7R,9Z)-18-({ [3-(dimethylamino)propyloxy]carbonyl}oxy)octacos-9-en-7-yl acetate having the following structural formulae:

[Formula 6]

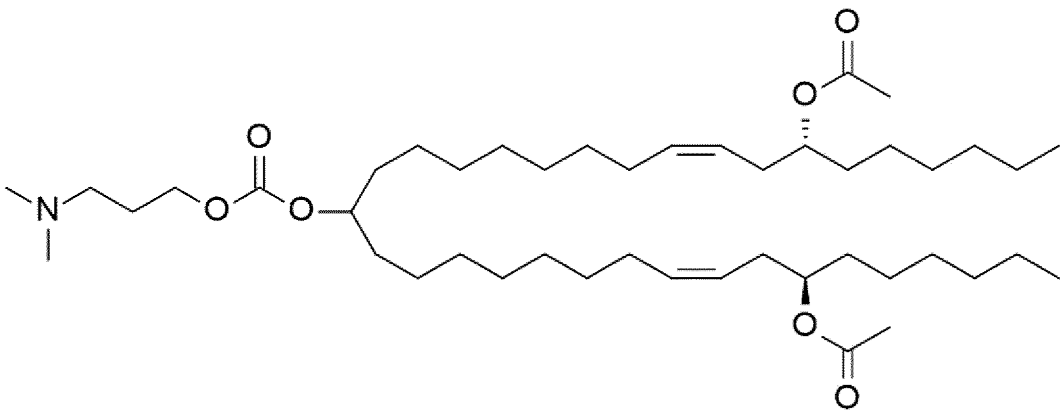

[Formula 7]

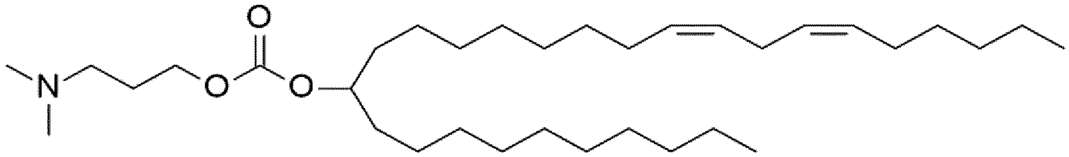

[Formula 8]

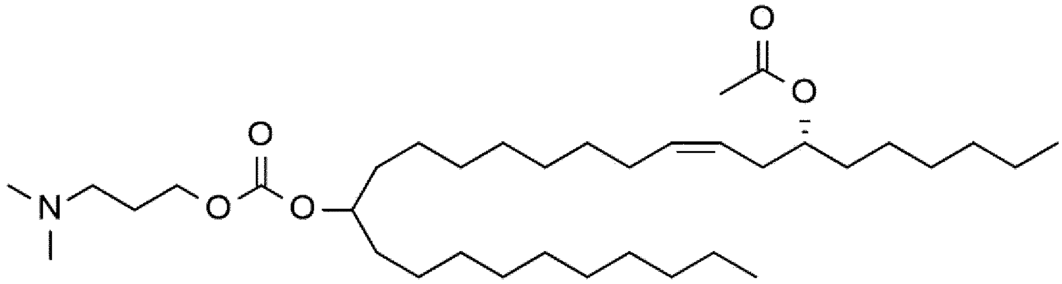

, respectively, are examples of cationic lipids which are components forming the particles of the present invention.

The cationic lipid having the general formula (Ia) may be one type of compound, or a combination of two or more types of compounds.

A method for producing a cationic lipid having the general formula (Ia) is described in International Publication No. WO 2015/005253.

The lipid according to the present invention may further comprise an amphipathic lipid, a sterol and a PEG lipid.

The amphipathic lipid has affinity for both polar and non-polar solvents, and specifically, distearoyl phosphatidylcholine, dioleoyl phosphatidylcholine, dioleoyl phosphatidylethanolamine, a combination thereof or the like are examples of amphipathic lipids. The amphipathic lipid used for the particles of the present invention is preferably distearoyl phosphatidylcholine and/or dioleoyl phosphatidylethanolamine, more preferably distearoyl phosphatidylcholine.

The sterol is a sterol having a hydroxy group, and specifically, cholesterol or the like can be exemplified.

The PEG lipid is a lipid modified with PEG, and specifically, 1,2-dimyristoyl-sn-glycelol methoxypolyethylene glycol and/or N-[methoxy poly(ethylene glycol) 2000]carbamoyl]-1,2-dimyristyloxypropyl-3-amine, a combination thereof, or the like can be exemplified, with 1,2-dimyristoyl-sn-glycelol methoxypolyethylene glycol being preferable. The average molecular weight of PEG lipid is not particularly limited, and is, for example, 1,000 to 5,000, preferably 1,500 to 3,000, more preferably 1,800 to 2,200.

The lipid composition of the amphipathic lipid, the sterol, the cationic lipid and the PEG lipid is not particularly limited, and is, for example, amphipathic lipid: 5 to 25%, sterol: 10 to 55%, cationic lipid: 40 to 65% and PEG lipid: 1 to 5% on a molar amount basis. Preferably, the lipid composition of the amphipathic lipid, the sterol, the cationic lipid and the PEG lipid is amphipathic lipid: 10 to 25%, sterol: 10 to 55%, cationic lipid: 40 to 65% and PEG lipid: 1 to 5% on a molar amount basis. More preferably, the lipid composition of the amphipathic lipid, the sterol, the cationic lipid and the PEG lipid is amphipathic lipid: 10 to 22.5%, sterol: 15 to 55%, cationic lipid: 40 to 65% and PEG lipid: 1 to 5% on a molar amount basis. In the lipid composition, the proportion of the PEG lipid is more preferably 1 to 3%, further more preferably 1 to 2%, further more preferably 1.2 to 2%, further more preferably 1.25 to 2%, further more preferably 1.3 to 2%, further more preferably 1.5 to 2% on a molar amount basis. In the lipid composition, the ratio of the total weight of lipids to the weight of the nucleic acid is not particularly limited, may be from 15 to 30, and is preferably from 15 to 25, more preferably from 15 to 22.5, further more preferably from 17.5 to 22.5.

When 3-dimethylaminopropyl(9Z,12Z)-octaoctacosa-19,22-dien-11-yl carbonate or (7R,9Z,26Z,29R)-18-({[3-(dimethylamino)propoxy]carbonyl}oxy)pentatriaconta-9,26-diene-7,29-diyl diacetate is used as the cationic lipid, the lipid composition of the amphipathic lipid, the sterol, the cationic lipid and the PEG lipid is not particularly limited, and is, for example, amphipathic lipid: 5 to 25%, sterol: 10 to 55%, cationic lipid: 40 to 65% and PEG lipid: 1 to 5%, preferably amphipathic lipid: 5 to 15%, sterol: 20 to 55%, cationic lipid: 40 to 65% and PEG lipid: 1 to 5%, on a molar amount basis. More preferably, the lipid composition of the amphipathic lipid, the sterol, the cationic lipid and the PEG lipid is amphipathic lipid: 5 to 15%, sterol: 35 to 50%, cationic lipid: 40 to 55% and PEG lipid: 1 to 3% on a molar amount basis. Further more preferably, the lipid composition of the amphipathic lipid, the sterol, the cationic lipid and the PEG lipid is amphipathic lipid: 10 to 15%, sterol: 35 to 45%, cationic lipid: 40 to 50% and PEG lipid: 1 to 2.5% on a molar

amount basis. Further more preferably, the lipid composition of the amphipathic lipid, the sterol, the cationic lipid and the PEG lipid is amphipathic lipid: 10 to 15%, sterol: 35 to 45%, cationic lipid: 40 to 50% and PEG lipid: 1 to 2% on a molar amount basis. In the lipid composition, the PEG lipid is more preferably 1.2 to 2%, further more preferably 1.25 to 2%, further more preferably 1.3 to 2%, further more preferably 1.5 to 2%. In the lipid composition, the ratio of the total weight of lipids to the weight of the nucleic acid is not particularly limited, may be from 15 to 30, and is preferably from 15 to 25, more preferably from 15 to 22.5, further more preferably from 17.5 to 22.5.

When (7R,9Z)-18-({ [3-(dimethylamino)propyloxy]carbonyl}oxy)octacos-9-en-7-yl acetate is used as the cationic lipid, the lipid composition of the amphipathic lipid, the sterol, the cationic lipid and the PEG lipid is not particularly limited, and is, for example, amphipathic lipid: 5 to 25%, sterol: 10 to 55%, cationic lipid: 40 to 65% and PEG lipid: 1 to 5%, preferably amphipathic lipid: 10 to 25%, sterol: 10 to 50%, cationic lipid: 40 to 65% and PEG lipid: 1 to 3%, on a molar amount basis. More preferably, the lipid composition of the amphipathic lipid, the sterol, the cationic lipid and the PEG lipid is amphipathic lipid: 10 to 25%, sterol: 10 to 45%, cationic lipid: 42.5 to 65% and PEG lipid: 1 to 2.5% on a molar amount basis. Further, more preferably, the lipid composition of the amphipathic lipid, the sterol, the cationic lipid and the PEG lipid is amphipathic lipid: 15 to 22.5%, sterol: 15 to 40%, cationic lipid: 45 to 65% and PEG lipid: 1 to 2% on a molar amount basis. Further, more preferably, the lipid composition of the amphipathic lipid, the sterol, the cationic lipid and the PEG lipid is amphipathic lipid: 17.5 to 22.5%, sterol: 15 to 40%, cationic lipid: 45 to 65% and PEG lipid: 1 to 2% on a molar amount basis. In the lipid composition, the PEG lipid is more preferably 1.2 to 2%, further, more preferably 1.25 to 2%, further, more preferably 1.3 to 2%, further, more preferably 1.5 to 2%. In the lipid composition, the ratio of the total weight of lipids to the weight of the nucleic acid is not particularly limited, may be from 15 to 30, and is preferably from 15 to 25, more preferably from 15 to 22.5, further, more preferably from 17.5 to 22.5.

As a specific combination of lipids in the present invention, distearoyl phosphatidylcholine, dioleoyl phosphatidylcholine or dioleoyl phosphatidylethanolamine as the amphipathic lipid, cholesterol as the sterol, (7R,9Z,26Z,29R)-18-({[3-(dimethylamino)propoxy]carbonyl}oxy)pentatriaconta-9,26-diene-7,29-diyl diacetate, 3-dimethylaminopropyl(9Z,12Z)-octaoctacosa-19,22-dien-11-yl carbonate or (7R,9Z)-18-({[3-(dimethylamino)propyloxy]carbonyl}oxy)octacos-9-en-7-yl acetate as the cationic lipid, and 1,2-dimyristoyl-sn-glycelolmethoxypolyethylene glycol or N-[methoxy poly(ethylene glycol) 2000]carbamoyl]-1,2-dimyristyloxypropyl-3-amine as the PEG lipid may be used in combination. A combination of distearoyl phosphatidylcholine or dioleoyl phosphatidylethanolamine as the amphipathic lipid, cholesterol as the sterol, (7R,9Z,26Z,29R)-18-({[3-(dimethylamino)propoxy]carbonyl}oxy)pentatriaconta-9,26-diene-7,29-diyl diacetate or (7R,9Z)-18-({[3-(dimethylamino)propyloxy]carbonyl}oxy)octacos-9-en-7-yl acetate as the cationic lipid and 1,2-dimyristoyl-sn-glycelol methoxypolyethylene glycol as the PEG lipid is preferable. The specific combination of lipids in the present invention is more preferably a combination of distearoyl phosphatidylcholine as the amphipathic lipid, cholesterol as the sterol, (7R,9Z,26Z,29R)-18-({[3-(dimethylamino)propoxy]carbonyl}oxy)pentatriaconta-9,26-diene-7,29-diyl diacetate or (7R,9Z)-18-({[3-(dimethylamino)propyloxy]carbonyl}oxy)octacos-9-en-7-yl acetate as the cationic lipid and 1,2-dimyristoyl-sn-glycelol methoxypolyethylene glycol as the PEG lipid.

In the present invention, the nucleic acid encapsulated in the lipid particles is capable of expressing the E6 antigen and the E7 antigen of human papillomavirus type 6 and/or type 11. The E6 antigen and the E7 antigen of human papillomavirus type 6 and/or type 11 which are expressed by the nucleic acid encapsulated in the lipid particles may be in the form of a fusion protein of the E6 antigen and the E7 antigen of human papillomavirus type 6, a fusion protein of the E6 antigen and the E7 antigen of human papillomavirus type 11, or a fusion protein of the E6 antigen and the E7 antigen of human papillomavirus type 6 and the E6 antigen and the E7 antigen of human papillomavirus type 11, and a protease cleavage sequence may be present between the neighboring antigens. The antigen may form a fusion protein with a signal peptide. Examples of the signal peptide for secreting the antigen into the extracellular space include an IgE leader sequence. Preferably, the signal peptide is fused to the N-terminal side of the antigen. For example, the fusion protein of the signal peptide and the antigen is an amino acid sequence of SEQ ID NO: 17, and the amino acid sequence extending from the 1st to 18th of the amino acid sequence of SEQ ID NO: 17 is the amino acid sequence of the IgE leader sequence.

The amino acid sequence of the E6 antigen of HPV type 6 is set forth as SEQ ID NO: 12. The nucleic acid encapsulated in the lipid particles encodes an E6 antigen of HPV type 6 which consists of an amino acid sequence having an identity of at least 95%, preferably 96%, more preferably 97% with the amino acid sequence of SEQ ID NO: 12.

The amino acid sequence of the E7 antigen of HPV type 6 is set forth as SEQ ID NO: 13. The nucleic acid encapsulated in the lipid particles encodes an E7 antigen of HPV type 6 which consists of an amino acid sequence having an identity of at least 95%, preferably 96%, more preferably 97% with the amino acid sequence of SEQ ID NO: 13.

The amino acid sequence of the E6 antigen of HPV type 11 is set forth as SEQ ID NO: 14. The nucleic acid encapsulated in the lipid particles encodes an E6 antigen of HPV type 11 which consists of an amino acid sequence having an identity of at least 95%, preferably 96%, more preferably 97% with the amino acid sequence of SEQ ID NO: 14.

The amino acid sequence of the E7 antigen of HPV type 11 is set forth as SEQ ID NO: 15. The nucleic acid encapsulated in the lipid particles encodes an E7 antigen of HPV type 11 which consists of an amino acid sequence having an identity of at least 95%, preferably 96%, more preferably 97% with the amino acid sequence of SEQ ID NO: 15.

The amino acid sequence of the protease cleavage sequence (furin cleavage site) is set forth as SEQ ID NO: 16. The protease cleavage sequence is a sequence that is cleaved by furin protein, and examples thereof include sequences represented by R-X-K/R-R (R represents arginine, K represents lysin, and X represents an arbitrary amino acid) (J. Biol. Chem. 1992, 267, 16396; J. Biol. Chem. 1991, 266, 12127).

The amino acid sequence of the fusion protein of the E6 antigen and the E7 antigen of HPV type 6 is set forth as SEQ ID NO: 17. The nucleic acid encapsulated in the lipid particles encodes a fusion protein of an E6 antigen and an E7 antigen of HPV type 6, which consists of an amino acid sequence having an identity of at least 95%, preferably 96%, more preferably 97% with the amino acid sequence of SEQ ID NO: 17.

The amino acid sequence of the fusion protein of the E6 antigen and the E7 antigen of HPV type 11 is set forth as SEQ ID NO: 18. The nucleic acid encapsulated in the lipid particles encodes a fusion protein of an E6 antigen and an E7 antigen of HPV type 11, which consists of an amino acid sequence having an identity of at least 95%, preferably 96%, more preferably 97% with the amino acid sequence of SEQ ID NO: 18.

The amino acid sequence of the fusion protein of the E6 antigen and the E7 antigen of HPV type 6 and the E6 antigen and the E7 antigen of HPV type 11 is set forth as SEQ ID NO: 19. The nucleic acid encapsulated in the lipid particles encodes a fusion protein of an E6 antigen and an E7 antigen of HPV type 6 and an E6 antigen and an E7 antigen of HPV type 11, which consists of an amino acid sequence having an identity of at least 95%, preferably 96%, more preferably 97% with the amino acid sequence of SEQ ID NO: 19.

The identity of amino acid sequences is a quantified ratio of matched amino acids with respect to the full-length sequence where amino acids are considered identical to corresponding amino acids when completely matched therewith. The identity of the sequence in the present invention is calculated using sequence analysis software GENETYX-SV/RC (manufactured by GENETYX Corporation), and its algorithm is commonly used in the art. Amino acids encoded by the nucleic acid encapsulated in the lipid particles of the present invention may undergo mutation(substitution), deletion, insertion and/or addition of amino acids as long as the amino acids maintain a certain level of identity with SEQ ID NOS: 12 to 20.

The amino acids encoded by the nucleic acid encapsulated in the lipid particles of the present invention maintain a sequence identity described above, and at several (preferably 5 or less, more preferably 3, 2 or 1) positions in each of the amino acid sequences of SEQ ID NOS: 12 to 20, several (preferably 10 or less, more preferably 7 or less, further more preferably 5, 4, 3, 2 or 1) amino acids per position may be substituted, deleted, inserted and/or added.

The nucleic acid capable of expressing the E6 antigen and the E7 antigen of human papillomavirus type 6 and/or the E6 antigen and the E7 antigen of human papillomavirus type 11 is an mRNA comprising a cap structure (Cap), a 5' untranslated region (5'-UTR), a leader sequence, a translated region of E6, a protease cleavage sequence (furin cleavage site), a translated region of E7, a 3' untranslated region (3'-UTR) and a poly A tail (polyA). The cap structure (Cap) is a site which is present at the 5'-end of mRNA in many eukaryotes and has a 7-methylguanosine structure. Examples of the cap structure include cap structures associated with the use of cap 0, cap 1, cap 2 or ARCA (Anti-reverse Cap Analog). The cap structures have the structural formulae shown below.

[Formula 9]

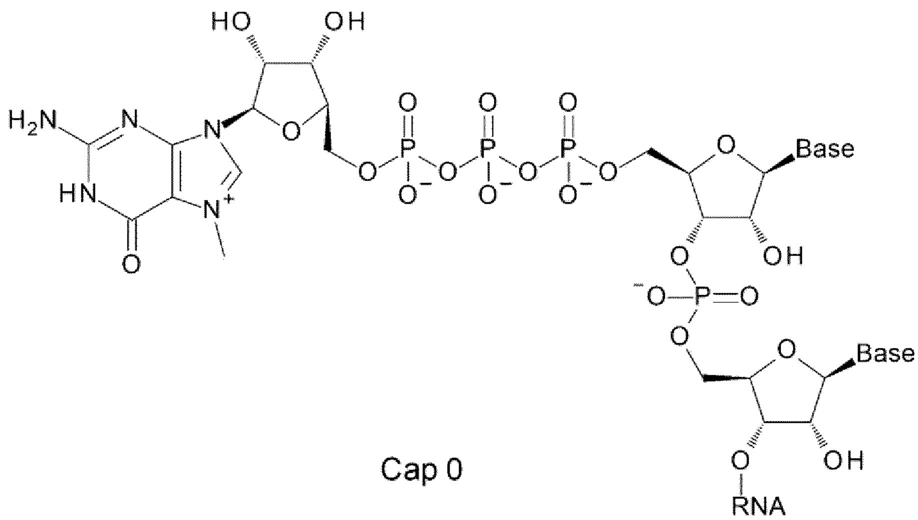

Cap 0

wherein Base represents any unmodified or modified nucleic acid base, and RNA represents any polynucleotide.

[Formula 10]

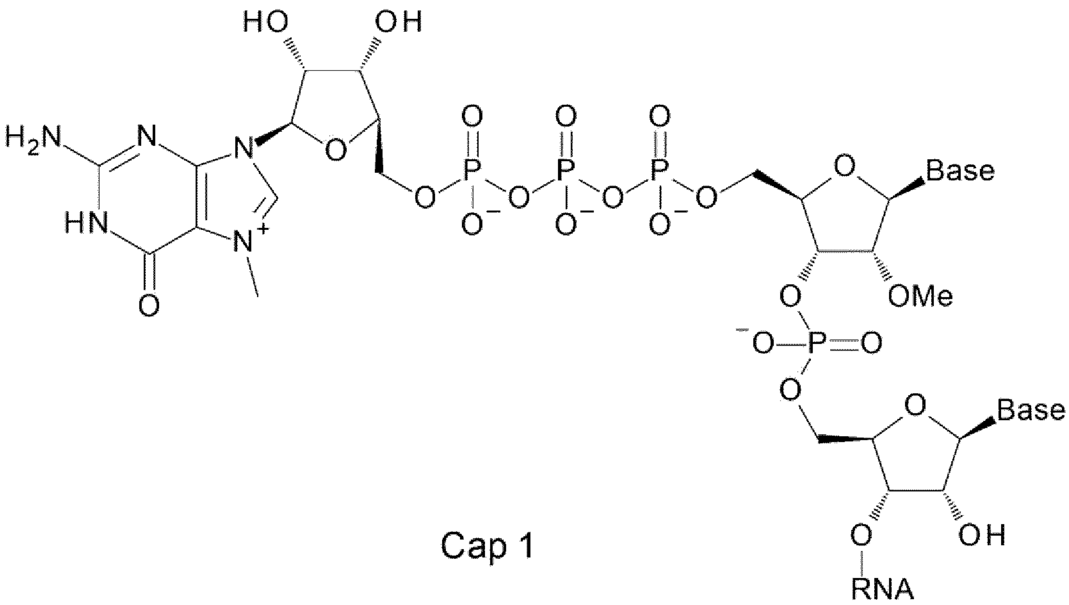

Cap 1

wherein Base represents any unmodified or modified nucleic acid base, and RNA represents any polynucleotide.

[Formula 11]

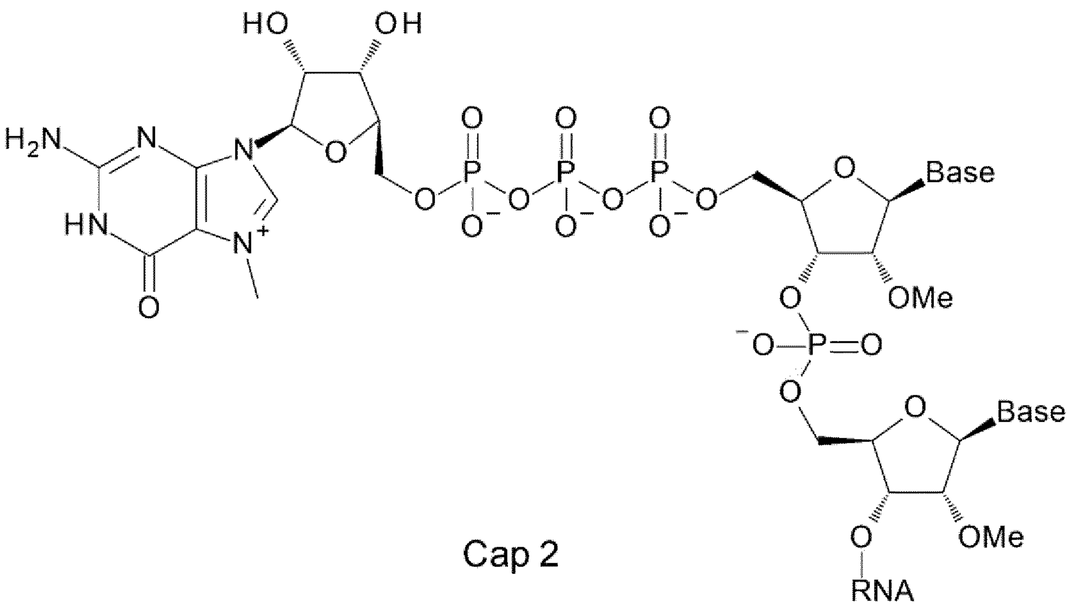

Cap 2

wherein Base represents any unmodified or modified nucleic acid base, and RNA represents any polynucleotide.

[Formula 12]

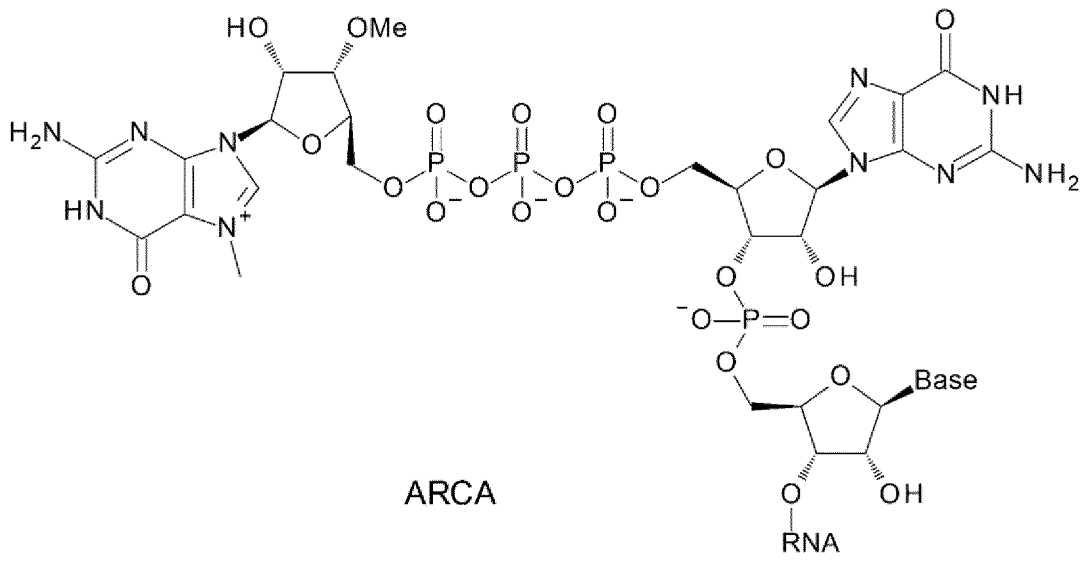

ARCA

wherein Base represents any unmodified or modified nucleic acid base, and RNA represents any polynucleotide.

The cap structure of mRNA in the present invention is preferably cap 0 or cap 1, more preferably cap 1.

The sequences of the 5' untranslated region and the 3' untranslated region are not particularly limited, and an untranslated region of stable mRNA such as α-globin, β-globin, actin or GAPDH can be used. The untranslated region used for the nucleic acid encapsulated in the lipid particles of the present invention is preferably an untranslated region of β-globin. For example, a sequence comprising base Nos. 15 to 64 in the sequence of SEQ ID NO: 2 can be used as a 5' untranslated region of β-globin, and a sequence comprising base Nos. 887 to 1018 in the sequence of SEQ ID NO: 2 may be used as a 3' untranslated region of β-globin.

Examples of the sequence of the 5' untranslated region (5'-UTR) include the sequence of base Nos. 1 to 70 in the sequence of SEQ ID NO: 5, the sequence of base Nos. 1 to 70 in the sequence of SEQ ID NO: 8, and the sequence of base Nos. 1 to 70 in the sequence of SEQ ID NO: 11.

Examples of the sequence of the leader sequence include the sequence of base Nos. 71 to 124 in the sequence of SEQ ID NO: 5, the sequence of base Nos. 71 to 124 in the sequence of SEQ ID NO: 8, and the sequence of base Nos. 71 to 124 in the sequence of SEQ ID NO: 11.

The sequence of the translated region of E6 is capable of expressing the whole or a part of the amino acid sequence of the E6 antigen and may comprise a start codon and/or a stop codon. Examples thereof include the sequence of base Nos. 125 to 571 in the sequence of SEQ ID NO: 5, the sequence of base Nos. 125 to 571 in the sequence of SEQ ID NO: 8, and the sequences of base Nos. 125 to 571 and 905 to 1351 in the sequence of SEQ ID NO: 11.

Examples of the sequence of the protease cleavage sequence (furin cleavage site) include the sequence of base Nos. 572 to 592 in the sequence of SEQ ID NO: 5, the sequence of base Nos. 572 to 592 in the sequence of SEQ ID NO: 8, and the sequences of base Nos. 572 to 592, 884 to 904 and 1352 to 1372 in the sequence of SEQ ID NO: 11.

The sequence of the translated region of E7 is capable of expressing the whole or a part of the amino acid sequence of the E7 antigen and may comprise a start codon and/or a stop codon. Examples thereof include the sequence of base Nos. 593 to 886 in the sequence of SEQ ID NO: 5, the sequence of base Nos. 593 to 886 in the sequence of SEQ ID NO: 8, and the sequences of base Nos. 593 to 883 and 1373 to 1666 in the sequence of SEQ ID NO: 11.

Examples of the sequence of the untranslated region (3'-UTR) include the sequence of base Nos. 887 to 1018 in the sequence of SEQ ID NO: 5, the sequence of base Nos. 887 to 1018 in the sequence of SEQ ID NO: 8, and the sequence of base Nos. 1667 to 1798 in the sequence of SEQ ID NO: 11.

Examples of the sequence of the poly A tail (polyA) include the sequence of base Nos. 1019 to 1118 in the sequence of SEQ ID NO: 5, the sequence of base Nos. 1019 to 1118 in the sequence of SEQ ID NO: 8, and the sequence of base Nos. 1799 to 1898.

The sequences of the cap structure (Cap), the 5' untranslated region (5'-UTR), the leader sequence, the translated region of E6, the protease cleavage sequence (furin cleavage site), the translated region of E7, the 3' untranslated region (3'-UTR) and the poly A tail (polyA) may be altered, and the sequence of the nucleic acid capable of expressing the E6 antigen and the E7 antigen of HPV type 6 may consist of a nucleotide sequence having an identity of at least 90%, preferably 95%, more preferably 97% with the sequence of SEQ ID NO: 5. The sequence of the nucleic acid capable of expressing the E6 antigen and the E7 antigen of HPV type 11 may consist of a nucleotide sequence having an identity of at least 90%, preferably 95%, more preferably 97% with the sequence of SEQ ID NO: 11. Further, the sequence of the nucleic acid capable of expressing the E6 antigen and the E7 antigen of HPV type 6 and type 11

may consist of a nucleotide sequence having an identity of at least 90%, preferably 95%, more preferably 97% with the sequence of SEQ ID NO: 11.

The length of the poly A tail is not particularly limited, and is, for example, a length of 10 to 250 bases, preferably a length of 15 to 120 bases, more preferably a length of 15 to 115 bases, particularly preferably a length of 20 to 110 bases.

The mRNA according to the present invention may be an mRNA consisting of a nucleotide sequence in which the sequence comprises a cap structure (Cap), a 5' untranslated region (5'-UTR), a translated region of E6, a protease cleavage sequence (furin cleavage site), a translated region of E7 and a 3' untranslated region (3'-UTR), and a part consisting of the cap structure (Cap), the 5' untranslated region (5'-UTR), the translated region of E6, the protease cleavage sequence (furin cleavage site), the translated region of E7 and the 3' untranslated region (3'-UTR) has an identity of at least 90%, preferably 95%, more preferably 97% with the sequence of residues 1 to 1018 in SEQ ID NO: 5, the sequence of residues 1 to 1018 in SEQ ID NO: 8 or the sequence of residues 1 to 1798 in SEQ ID NO: 11.

The nucleic acid encapsulated in the lipid particles may be in any form as long as it is capable of expressing the E6 antigen and the E7 antigen of human papillomavirus type 6 and/or type 11. Examples thereof include single-stranded DNA, single-stranded RNA (for example, mRNA), single-stranded polynucleotide in which DNA and RNA are mixed, double-stranded DNA, double-stranded RNA, hybrid polynucleotide of DNA-RNA, and double-stranded polynucleotide comprising two polynucleotides in which DNA and RNA are mixed. mRNA is preferred.

The nucleotide forming a nucleic acid encapsulated in the lipid particles may be natural or modified nucleotide, and at least one modified nucleotide is preferably included.

The modified part in the modified nucleotide may be any of a base, a sugar and a phosphoric acid diester bond. There may be one or more modification sites.

Examples of the modification of a base include 5-methylation of cytosine, 5-fluoridation, N4-methylation, 5-methylation of uracil (thymine), 5-fluoridation, N6-methylation of adenine, and N2-methylation of guanine.

Examples of the modification of sugar include 2'-O-methylation of D-ribofuranose.

Examples of the modification of a phosphoric acid diester bond include phosphorothioate bond.

The modified nucleotide is preferably one that is modified at a base part. Examples thereof include pyrimidine nucleotide substituted at the 5-position, and pseudouridine optionally substituted at the 1-position. Specifically, 5-methylcytidine, 5-methoxyuridine, 5-methyluridine, pseudouridine, 1-alkylpseudouridine can be exemplified. The 1-alkylpseudouridine may be 1-(C1-C6 alkyl)pseudouridine, and is preferably 1-methylpseudouridine or 1-ethylpsedouridine. Examples of the more preferred modified nucleotide include 5-methylcytidine, 5-methyluridine, and 1-methylpseudouridine. Examples of the particularly preferred modified nucleotide include a combination of 5-methylcytidine and 5-methyluridine, and a combination of 5-methylcytidine and 1-methylpseudouridine.

The nucleic acid capable of expressing the E6 antigen and the E7 antigen of human papillomavirus (for example, HPV type 6 and HPV type 11) according to the present invention can be produced by an *in vitro* transcription reaction from DNA having a desired base sequence. An enzyme, a buffer solution and a nucleoside-5'-triphosphoric acid mixture (adenosine-5'-triphosphoric acid (ATP), guanosine-5'-triphosphoric acid (GTP), cytidine-5'-triphosphoric acid (CTP) and uridine-5'-triphosphoric acid (UTP)) that are necessary for *in vitro* transcription are commercially available (AmpliScribeT7 High Yield Transcription Kit (Epicentre), or mMESSAGE mMACHINE T7 Ultra Kit (Life technologies). DNA for use in production of single-stranded RNA is cloned DNA, for example, plasmid DNA or a DNA fragment. The plasmid DNA or DNA fragment used may be a commercial product or may be produced by a method generally known in the art (for example, a method described in Sambrook, J. et al., Molecular Cloning a Laboratory Manual second edition (1989), Rashtchian, A., Current Opinion in Biotechnology, 1995, 6(1), 30-36, Gibson D.G. et al., Science, 2008, 319 (5867), 1215-1220).

For obtaining mRNA having improved stability and/or safety, some or all of the unmodified nucleotides in the mRNA can also be replaced with modified nucleotides by replacing some or all of the unmodified nucleoside-5'-triphosphoric acids with modified nucleoside-5'-triphosphoric acids in the *in vitro* transcription reaction (Kormann, M., Nature Biotechnology, 2011, 29, 154-157.).

For obtaining mRNA having improved stability and/or safety, a cap structure (the Cap 0 structure described above) can be introduced to the 5'-end of mRNA by a method in which a capping enzyme is used after the *in vitro* transcription reaction. Further, Cap 0 can be converted into Cap 1 by a method in which 2'-O-methyltransferase is applied to mRNA having Cap 0. The capping enzyme and the 2'-O-methyltransefrase used may be commercial products (for example, Vaccinia Capping System, N2080; mRNA Cap 2'-O-Methyltransferase, M0366 both manufactured by New England Biolab, Inc.). When a commercial product is used, mRNA having a cap structure can be produced in accordance with a protocol accompanying the product.

The cap structure at the 5'-end of mRNA can also be introduced by a method other than a method in which an enzyme is used. For example, a structure of a cap analog of ARCA or a Cap 1 structure derived from CleanCap (registered trademark) can be introduced into mRNA by adding ARCA or CleanCap (registered trademark) to the *in vitro* transcription reaction. Commercially available products can be used for ARCA and CleanCap (registered trademark) (ARCA, N-7003; CleanCap Reagent AG, N-7113 both manufactured by TriLink BioTechnologies, LLC). When a commercially available

product is used, mRNA having a cap structure can be produced in accordance with a protocol accompanying the product.

In the present invention, the nucleic acid encapsulated in the lipid particles may be purified by any a method such as desalting, HPLC (reverse phase, gel permeation, ion exchange, affinity), PAGE or ultrafiltration. Removal of impurities by purification treatment can reduce production of inflammatory cytokines in a living body receiving the nucleic acid.

The lipid particles encapsulating a nucleic acid according to the present invention can be produced by a method such as a thin-film method, a reverse phase evaporation method, an ethanol injection method, an ether injection method, a dehydration-rehydration method, a surfactant dialysis method, a hydration method or a freezing and thawing method. The lipid particles encapsulating a nucleic acid can be produced by, for example, a method disclosed in International Publication No. WO 2015/005253. The lipid particles encapsulating a nucleic acid of the present invention can also be produced by mixing a nucleic acid solution and a lipid solution in a microchannel. For example, using NanoAssemblr (registered trademark) from Precision Nanosystems Inc., the lipid particles encapsulating a nucleic acid can be produced by the method described in the accompanying protocol.

The particles of the present invention have an average particle size of 30 to 300 nm, preferably 30 to 200 nm, more preferably 30 to 100 nm. The average particle size can be obtained by measuring a volume average particle size on the basis of the principle of a dynamic light scattering method or the like using equipment such as Zeta Potential/Particle Sizer NICOMP (registered trademark) 380ZLS (PARTICLE SIZING SYSTEMS).

The particles of the present invention may be used for producing a composition for preventing and/or treating a disease caused by infection with human papillomavirus type 6 and/or type 11 (recurrent respiratory papillomatosis, condyloma acuminatum, or the like).

The E6 antigen and the E7 antigen of human papillomavirus type 6 and/or type 11 can be expressed *in vivo* or *in vitro* using the particles of the present invention. Accordingly, the present invention provides a method for expressing the E6 antigen and the E7 antigen of human papillomavirus type 6 and/or type 11 *in vitro,* comprising introducing a composition containing the particles into cells. The present invention also provides a method for expressing the E6 antigen and the E7 antigen of human papillomavirus type 6 and/or type 11 *in vivo,* comprising administering a composition containing the particles to a mammal. By expressing the E6 antigen and the E7 antigen of human papillomavirus type 6 and/or type 11 *in vivo,* an immune reaction against human papillomavirus type 6 and/or type 11 can be induced. As a result, infection with human papillomavirus type 6 and/or type 11 can be prevented and/or treated. Accordingly, the present invention provides a method for inducing an immune reaction against human papillomavirus type 6 and/or type 11, comprising administering a composition containing the particles to a mammal. The present invention provides a method for preventing and/or treating infection with human papillomavirus type 6 and/or type 11, comprising administering a composition containing the particles to a mammal.

The particles of the present invention can be used as a medicament and as a laboratory reagent. The particles of the present invention are typically added to a carrier such as water, a buffer solution or physiological saline. The resulting formulation (composition) can be introduced into cells (*in vitro*), or administered to a mammal (*in vivo*). In the case of administration to a mammal, the carrier is a pharmaceutically acceptable carrier (for example, physiological saline). The particles of the present invention may be formulated into a dosage form such as a cream, a paste, an ointment, a gel or a lotion made using fat, fatty oil, lanolin, vaseline, paraffin, wax, resin, plastic, glycol, a higher alcohol, glycerin, water, an emulsifier, a suspension agent or the like as a substrate material.

The particles of the present invention can be administered to mammals such as humans, mice, rats, hamsters, guinea pigs, rabbits, pigs, monkeys, cats, dogs, horses, goats, sheep and bovines orally, or parenterally by a method such as intramuscular administration, intravenous administration, intrarectal administration, transdermal administration, transmucosal administration, subcutaneous administration or intracutaneous administration.

When the particles of the present invention are administered to a human, for example, the particles are intramuscularly injected, subcutaneously injected, intracutaneously injected, drip-injected intravenously, or intravenously injected once or several times in a dosage amount of about 0.001 to 1 mg, preferably 0.01 to 0.2 mg of mRNA per administration per adult, but the dosage amount and the frequency of administration can be appropriately changed depending on the type of disease, the symptom, the age and the administration method.

When the particles of the present invention are used as a laboratory reagent, the E6 antigen and the E7 antigen of human papillomavirus type 6 and/or type 11 can be expressed *in vitro* by introducing the particles into cells in which the E6 antigen and the E7 antigen of human papillomavirus type 6 and/or type 11 are to be expressed (for example, HEK 293 cells and derived cells thereof (HEK 293T cells, FreeStyle 293 cells and Expi 293 cells), CHO cells, C2C12 mouse myoblast cells, immortalized mouse dendritic cells (MutuDC1940)). Expression of the E6 antigen and the E7 antigen of human papillomavirus type 6 and/or type 11 can be analyzed by detecting the E6 antigen and the E7 antigen of human papillomavirus type 6 and/or type 11 in a sample by a Western-blot method, or detecting a peptide fragment specific to the E6 antigen and the E7 antigen of human papillomavirus type 6 and/or type 11 by mass spectrometry.

The term "treatment" as used herein means that in a patient having developed an infection with a virus, bacteria or the like, or a disease caused by the infection (for example, recurrent respiratory papillomatosis, condyloma acuminatum,

precancerous lesion or cancer), the clinical symptoms of such a disease are cured, caused to remit, alleviated and/or delayed from worsening.

The term "prevention" as used herein means reducing incidence of a disease caused by an infection with a virus, bacteria or the like. The prevention includes lowering the risk of progression of a disease caused by an infection with a virus, bacteria or the like, or reducing aggravation of such a disease. The particles of the present invention are effective in prevention and/or treatment of the disease because they induce a preventive immune reaction.

Examples

Hereinafter, the present invention will be described in detail by way of Examples, which are intended to illustrate the present invention, and should not be construed as limiting the scope of the present invention.

[Example 1]

Preparation of HPV6 E6-E7 fusion mRNA-001

(1) Production of template DNA for *in vitro* transcription (IVT) of HPV6 E6-E7 fusion

A plasmid was constructed for producing template DNA for use in *in vitro* transcription (IVT). Specifically, a plasmid (pMA-HPV6) was produced that contains a DNA fragment (SEQ ID NO: 1) comprising a sequence in which GCTAGC (NheI site), a T7 promoter sequence, a 5'-UTR sequence of human β-globin, a KOZAK sequence, a translated region of IgE leader sequence-HPV type 6 E6-furin cleavage site-HPV type 6 E7, a 3'-UTR sequence of human β-globin, a poly A tail and ACTAGT (SpeI site) connected in this order. To nuclease-free water (566.4 μL) in which 8 ng of the plasmid treated with SpeI, a restriction enzyme, 10 × Buffer for KOD-Plus- Ver. 2 (80 μL, Toyobo Co., Ltd. catalog # KOD-211), 2 mM dNTP mix (80 μL, Toyobo Co., Ltd. catalog # KOD-211), 25 mM MgSO4 (48 μL, Toyobo Co., Ltd. catalog # KOD-211), a 50 μM sense primer (4.8 μL, SEQ ID NO: 2), a 50 μM antisense primer (4.8 μL, SEQ ID NO: 3), and KOD Plus polymerase (16 μL, Toyobo Co., Ltd. catalog # KOD-211) were added. The mixture was incubated at 98°C for 1 minute, incubated at 98°C for 5 seconds, at 55°C for 15 seconds and at 68°C for 2 minutes 20 times, and further incubated at 68°C for 1 minute to amplify HPV6 E6-E7 fusion DNA. After the reaction, template DNA (SEQ ID NO: 4) was purified with Wizard SV Gel and PCR Clean-Up System (Promega catalog # A9281).

(2) Preparation of HPV6 E6-E7 fusion mRNA-001 by *in vitro* transcription

The 479 μg/mL template DNA (57 μL) obtained in Example 1-(1), 100 mM CleanCap AG (55 μL, TriLink catalog # T-7113), 100 mM ATP (55 μL, Hongene catalog # R1331), 100 mM GTP (55 μL, Hongene catalog # R2331), 100 mM 5-methylcytidine-5'-triphosphate (55 μL, Hongene catalog # R3-029), 100 mM 5-methyluridine-5'-triphosphate (55 μL), Nuclease-free water (438 μL, Qiagen catalog # 129114), T7 Transcription 5× buffer (220 μL, Promega catalog # P140X), an enzyme mix and T7 RNA Polymerase (110 μL, Promega catalog # P137X) were mixed, and incubated at 37°C for 4 hours. RQ1 RNase-free DNase (27.5 μL, Promega catalog # M6101) was mixed, and the mixture was incubated at 37°C for 15 minutes. An 8 M LiCl solution (550 μL, Sigma-Aldrich catalog # L7026) was mixed, and the mixture was stored overnight at -20°C, and centrifuged (4°C, 4,000×g, 30 min). The supernatant was then discarded, 70% ethanol was added, the mixture was centrifuged (4°C, 4,000×g, 10 min), and the supernatant was then discarded, followed by drying in air. The obtained residue was dissolved in nuclease-free water, and the solution was then purified by using RNeasy Maxi kit (Qiagen catalog # 75162) in accordance with the attached manual. The obtained eluate (3 mL, 4,809 μg as measured by UV), nuclease-free water (88 μL), a buffer solution of rApid Alkaline Phosphatase (Roche catalog # 04 898 141 001) (450 μL) and an enzyme (962 μL) were mixed, incubated at 37°C for 30 minutes, and purified by using RNeasy Maxi kit in accordance with the attached manual, thereby obtaining the desired mRNA (3 mL, 4.1 mg as measured by UV) .

The obtained mRNA has the sequence of SEQ ID NO: 5. The mRNA was analyzed with LabChip GX Touch Standard RNA Reagent Kit (PerkinElmer catalog # CLS960010) and confirmed to have the desired length.

[Example 2]

Preparation of HPV11 E6-E7 fusion mRNA-002

(1) Production of template DNA for ITV of HPV11 E6-E7 fusion

A plasmid was constructed for producing template DNA for use as an IVT template. Specifically, a plasmid

(pMA-HPV11) was produced that contains a DNA fragment (SEQ ID NO: 6) comprising a sequence in which GCTAGC (NheI site), a T7 promoter sequence, a 5'-UTR sequence of human β-globin, a KOZAK sequence, a translated region of IgE leader sequence-HPV type 11 E6-furin cleavage site-HPV type 11 E7, a 3'-UTR sequence of human β-globin, a poly A tail and ACTAGT (SpeI site) connected in this order. To nuclease-free water (566.4 μL) in which 8 ng of the plasmid treated with SpeI, a restriction enzyme, 10 × Buffer for KOD-Plus- Ver. 2 (80 μL, Toyobo Co., Ltd. catalog # KOD-211), 2 mM dNTP mix (80 μL, Toyobo Co., Ltd. catalog # KOD-211), 25 mM MgSO4 (48 μL, Toyobo Co., Ltd. catalog # KOD-211), a 50 μM sense primer (4.8 μL, SEQ ID NO: 2), a 50 μM antisense primer (4.8 μL, SEQ ID NO: 3) and KOD Plus polymerase (16 μL, Toyobo Co., Ltd. catalog # KOD-211) were added. The mixture was incubated at 98°C for 1 minute, followed by 20 cycles of incubation at 98°C for 5 seconds, at 55°C for 15 seconds and at 68°C for 2 minutes, followed by a further incubation at 68°C for 1 minute to amplify HPV6 E6-E7 DNA. After the reaction, template DNA (SEQ ID NO: 7) was purified with Wizard SV Gel and PCR Clean-Up System (Promega catalog # A9281).

(2) Preparation of HPV11 E6-E7 fusion mRNA-002 by *in vitro* transcription

The 467 μg/mL template DNA (59 μL) obtained in Example 1-(1), 100 mM CleanCap AG (55 μL, TriLink catalog # T-7113), 100 mM ATP (55 μL, Hongene catalog # R1331), 100 mM GTP (55 μL, Hongene catalog # R2331), 100 mM 5-methylcytidine-5'-triphosphate (55 μL, Hongene catalog # R3-029), 100 mM 5-methyluridine-5'-triphosphate (55 μL), Nuclease-free water (436 μL, Qiagen catalog # 129114), T7 Transcription 5× buffer (220 μL, Promega catalog # P140X), an enzyme mix and T7 RNA Polymerase (110 μL, Promega catalog # P137X) were mixed, and incubated at 37°C for 4 hours. RQ1 RNase-free DNase (27.5 μL, Promega catalog # M6101) was mixed, and the mixture was incubated at 37°C for 15 minutes. An 8 M LiCl solution (550 μL, Sigma-Aldrich catalog # L7026) was mixed, and the mixture was stored overnight at -20°C, and centrifuged (4°C, 4,000×g, 30 min). The supernatant was then discarded, 70% ethanol was added, the mixture was centrifuged (4°C, 4,000×g, 10 min), and the supernatant was then discarded, followed by drying in air. The obtained residue was dissolved in nuclease-free water, and the solution was then purified by using RNeasy Maxi kit (Qiagen catalog # 75162) in accordance with the attached manual. The obtained eluate (3 mL, 4,769 μg as measured by UV), nuclease-free water (96 μL), a buffer solution of rApid Alkaline Phosphatase (Roche catalog # 04 898 141 001) (450 μL) and an enzyme (954 μL) were mixed, incubated at 37°C for 30 minutes, and purified by using RNeasy Maxi kit in accordance with the attached manual, thereby obtaining desired mRNA (3 mL, 3.8 mg as measured by UV).

The obtained mRNA has the sequence of SEQ ID NO: 8. The mRNA was analyzed with LabChip GX Touch Standard RNA Reagent Kit (PerkinElmer catalog # CLS960010) and confirmed to have the desired length.

[Example 3]

Preparation of HPV6 E6-E7 HPV11 E6-E7 fusion mRNA-003

(1) Production of template DNA for IVT of HPV6 E6-E7 HPV11 E6-E7 fusion

A plasmid was constructed for producing template DNA for use as an IVT template. Specifically, a plasmid (pMA-HPV6_HPV11) was produced that contains a DNA fragment (SEQ ID NO: 9) comprising a sequence in which GCTAGC (NheI site), a T7 promoter sequence, a 5'-UTR sequence of human β-globin, a KOZAK sequence, a translated region of IgE leader sequence-HPV type 6 E6-furin cleavage site-HPV type 6 E7-furin cleavage site-HPV type 11 E6-furin cleavage site-HPV type 11 E7, a 3'-UTR sequence of β-globin, a poly A tail and ACTAGT (SpeI site) connected in this order. To nuclease-free water (566.4 μL) in which 8 ng of the plasmid treated with SpeI, a restriction enzyme, 10 × Buffer for KOD-Plus-Ver. 2 (80 μL, Toyobo Co., Ltd. catalog # KOD-211), 2 mM dNTP mix (80 μL, Toyobo Co., Ltd. catalog # KOD-211), 25 mM MgSO4 (48 μL, Toyobo Co., Ltd. catalog # KOD-211), a 50 μM sense primer (4.8 μL, SEQ ID NO: 2), a 50 μM antisense primer (4.8 μL, SEQ ID NO: 3) and KOD Plus polymerase (16 μL, Toyobo Co., Ltd. catalog # KOD-211) were added. The mixture was incubated at 98°C for 1 minute, followed by 20 cycles of incubation at 98°C for 5 seconds, at 55°C for 15 seconds and at 68°C for 2 minutes, followed by a further incubation at 68°C for 1 minute to amplify HPV6 E6-E7 DNA. After the reaction, template DNA (SEQ ID NO: 10) was purified with Wizard SV Gel and PCR Clean-Up System (Promega catalog # A9281).

(2) Preparation of HPV6 E6-E7 HPV11 E6-E7 fusion mRNA-003 by *in vitro* transcription

The 413 μg/mL template DNA (67 μL) obtained in Example 1-(1), 100 mM CleanCap AG (55 μL, TriLink catalog # T-7113), 100 mM ATP (55 μL, Hongene catalog # R1331), 100 mM GTP (55 μL, Hongene catalog # R2331), 100 mM 5-methylcytidine-5'-triphosphate (55 μL, Hongene catalog # R3-029), 100 mM 5-methyluridine-5'-triphosphate (55 μL), Nuclease-free water (428 μL, Qiagen catalog # 129114), T7 Transcription 5× buffer (220 μL, Promega catalog # P140X),

an enzyme mix and T7 RNA Polymerase (110 μL, Promega catalog # P137X) were mixed, and incubated at 37°C for 4 hours. RQ1 RNase-free DNase (27.5 μL, Promega catalog # M6101) was mixed, and the mixture was incubated at 37°C for 15 minutes. An 8 M LiCl solution (550 μL, Sigma-Aldrich catalog # L7026) was mixed, and the mixture was stored overnight at -20°C, and centrifuged (4°C, 4,000×g, 30 min). The supernatant was then discarded, 70% ethanol was added, the mixture was centrifuged (4°C, 4,000×g, 10 min), and the supernatant was then discarded, followed by drying in air. The obtained residue was dissolved in nuclease-free water, and the solution was then purified by using RNeasy Maxi kit (Qiagen catalog # 75162) in accordance with the attached manual. The obtained eluate (3 mL, 4,345 μg as measured by UV), nuclease-free water (181 μL), a buffer solution of rApid Alkaline Phosphatase (Roche catalog # 04 898 141 001) (450 μL) and an enzyme (869 μL) were mixed, incubated at 37°C for 30 minutes, and purified by using RNeasy Maxi kit in accordance with the attached manual, thereby obtaining the desired mRNA (3 mL, 3.8 mg as measured by UV) .

The obtained mRNA has the sequence of SEQ ID NO: 11. The mRNA was analyzed with LabChip GX Touch Standard RNA Reagent Kit (PerkinElmer catalog # CLS960010) and confirmed to have the desired length.

[Examples 4 to 12]

Preparation of mRNA-encapsulating nucleic acid lipid particles using mRNAs of Examples 1 to 3

(1) Preparation of mRNA-encapsulating nucleic acid lipid particles

Distearoyl phosphatidylcholine (1,2-distearoyl-sn-glycero-3-phosphocholine: hereinafter, referred to as DSPC, NOF CORPORATION), cholesterol (hereinafter, referred to as Chol, Sigma-Aldrich, Inc.), (7R,9Z)-18-({[3-(dimethylamino)propyloxy]carbonyl}oxy)octacos-9-en-7-yl acetate (compound described in WO 2015/005253, Example 28) (hereinafter, referred to as LP), and 1,2-dimyristoyl-sn-glycerol methoxypolyethylene glycol having a polyethylene glycol molecular weight of about 2,000 (1,2-dimyristoyl-sn-glycero-3-methoxypolyethylene glycol, hereinafter, referred to as PEG-DMG, NOF CORPORATION) were dissolved in ethanol at the molar ratio shown in Table 1 so that the total lipid concentration was 5 mM.

Meanwhile, the mRNAs obtained in Examples 1 to 3 were diluted with a citrate buffer solution (20 mM Citrate buffer, pH 4.0).

Using NanoAssemblr BenchTop (Precision Nanosystems Inc.), the lipid solution and the mRNA solution were mixed at a volume ratio of 1 : 3 and the total lipid-to-mRNA weight ratio shown in Table 1in a microchannel to obtain a crude dispersion liquid of nucleic acid lipid particles. The dispersion liquid of nucleic acid lipid particles was dialyzed with a buffer solution in an amount about 25 to 50 times the amount of the dispersion liquid (Float-A-Lyzer G2, MWCO: 1,000 kD, Spectra/Por) for 12 to 18 hours to remove ethanol, thereby obtaining a purified dispersion liquid of mRNA-encapsulating nucleic acid lipid particles.

The LP was synthesized in accordance with the method described in WO 2015/005253, Example 28.

(2) Evaluation of characteristics of mRNA-encapsulating nucleic acid lipid particles

The characteristics of the prepared dispersion liquid containing nucleic acid lipid particles were evaluated. Methods for evaluating respective characteristics will be described.

(2-1) mRNA encapsulation percentage

Using Quant-iT RiboGreen RNA Assay Kit (Invitrogen), the mRNA encapsulation percentage was measured in accordance with the attached document.

The amount of mRNA in the dispersion liquid of nucleic acid lipid particles was determined in the presence and absence of a 0.015% Triton X-100 surfactant, and the encapsulation percentage was calculated from the following expression:

{([amount of mRNA in the presence of surfactant] - [amount of mRNA in the absence of surfactant]) / [amount of mRNA in the presence of surfactant]} × 100 (%).

(2-2) Ratio between mRNA and lipid

The amount of mRNA in the dispersion liquid of nucleic acid lipid particles was measured by any of the following methods.
The dispersion liquid of nucleic acid lipid particles was diluted with 1.0% Triton X-100, and the amount of mRNA was measured by reverse phase chromatography (system: Agilent 1260 series, column: Bioshell A400 Protein C4 (10 cm × 4.6 mm, 3.4 μm) (SUPELCO), buffer A: 0.1 M triethylamine acetate (pH 7.0), buffer B: acetonitrile, (B%): 5-50% (0-15 min), flow rate: 1 mL/min, temperature: 70°C, detection: 260 nm) .
The amount of mRNA in the nucleic acid lipid particles was measured with an ultraviolet and visible spectrophotometer (LAMBDA™ 465 manufactured by PerkinElmer, Inc.) by diluting and dissolving the dispersion liquid of nucleic acid lipid particles to a solution with 90% methanol. The mRNA concentration was calculated from the following expression:

{[absorbance at 260 nm] - [absorbance at 350 nm]} × 40 × dilution ratio (μg/mL).

The amount of each lipid in the dispersion liquid of nucleic acid lipid particles was measured by reverse phase chromatography (System: DIONEX UltiMate 3000, column: XSelect CSH C18 (130 Å, 3.5 μm, 3.0 mn × 150 mm) (Waters catalog # 186005263), buffer A: 0.2% formic acid, buffer B: 0.2% formic acid, methanol, (B%): 75-100% (0-6 min), 100% (6-15 min), flow rate: 0.45 mL/min, temperature: 50°C, detection: Corona CAD (Charged Aerosol Detector)).
The ratio of the total amount of lipids to mRNA was calculated from the following expression:

[total lipid concentration]/[mRNA concentration] (wt/wt).

(2-3) Average particle size

The particle size of the nucleic acid lipid particle was measured by Zeta Potential/Particle Sizer NICOMPTM 380 ZLS (PARTICLE SIZING SYSTEMS). The average particle size in the table represents the volume average particle size, and the number following ± represents the deviation.
Table 2 shows the results of the evaluation of characteristics.

[Table 1]

| | m R N A | DSPC | Chol | LP | PEG-DMG | Lipids/mRNA (wt/wt) |
|---|---|---|---|---|---|---|
| **Example 4** | **Example 1** | 17.5% | 26% | 55% | 1.5% | 20 |
| **Example 5** | **Example 2** | 17.5% | 26% | 55% | 1.5% | 20 |
| **Example 6** | **Example 3** | 17.5% | 26% | 55% | 1.5% | 20 |
| **Example 7** | **Example 1** | 17.5% | 21% | 60% | 1.5% | 20 |
| **Example 8** | **Example 2** | 17.5% | 21% | 60% | 1.5% | 20 |
| **Example 9** | **Example 3** | 17.5% | 21% | 60% | 1.5% | 20 |
| **Example 10** | **Example 1** | 22.5% | 16% | 60% | 1.5% | 20 |
| **Example 11** | **Example 2** | 22.5% | 16% | 60% | 1.5% | 20 |
| **Example 12** | **Example 3** | 22.5% | 16% | 60% | 1.5% | 20 |

[Table 2]

| | **Encapsulation percentage** (%) | **Particle size** (nm) |
|---|---|---|
| **Example 4** | 99 | 115±16 |
| **Example 5** | 99 | 111±32 |

(continued)

| | Encapsulation percentage (%) | Particle size (nm) |
|---|---|---|
| Example 6 | 99 | 120±51 |
| Example 7 | 99 | 119±36 |
| Example 8 | 98 | 120±46 |
| Example 9 | 98 | 128±51 |
| Example 10 | 96 | 130±48 |
| Example 11 | 96 | 128±35 |
| Example 12 | 96 | 132±43 |

The above results reveal that in these nucleic acid lipid particles, 95% or more of mRNA is encapsulated in lipid particles, and the average particle size is about 110 nm to about 140 nm.

[Test Example 1]

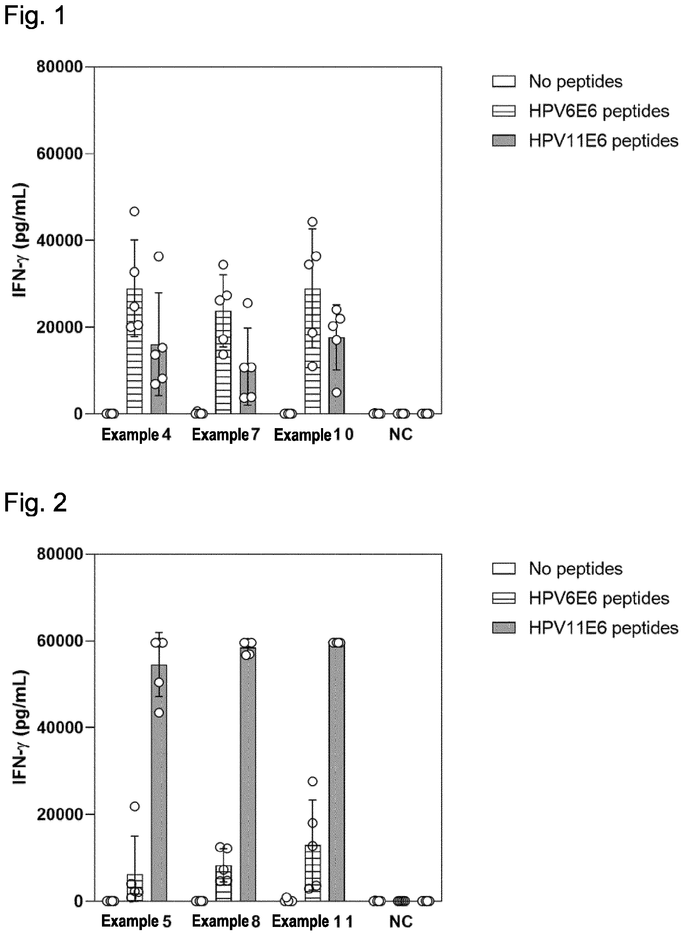

Ability to produce T cell cytokine specific to HPV genotype 6E6E7 vaccine antigen (Figure 1)

C57BL/6J mice were purchased from CLEA Japan, Inc. All treatments of the animals were performed under isoflurane inhalation anesthesia.

The mRNA-encapsulating nucleic acid lipid particles were administered into the gastrocnemius muscle of six-week-old C57BL/6 mice in an amount of 5 μg of mRNA per mouse twice at an interval of two weeks. One week after the final administration, the spleen was harvested, and spleen cells were prepared. The spleen cells were treated with a HPV6E6 pool peptide (manufactured by JPT Peptide Technologies, catalog # PM-HPV06-E6) or a HPV11E6 pool peptide (manufactured by JPT Peptide Technologies, catalog # PM-HPV11-E6), the culture supernatant after culturing for 48 hours was diluted by 2-fold or 30-fold, and the amount of IFN-γ was measured by a cytokine ELISA method.

[Test Example 2]

Ability to produce T cell cytokine specific to HPV genotype 11E6E7 vaccine antigen (Figure 2)

The mRNA-encapsulating nucleic acid lipid particles were administered into the gastrocnemius muscle of six-week-old C57BL/6 mice in an amount of 5 μg of mRNA per mouse twice at an interval of two weeks. One week after the final administration, the spleen was harvested, and spleen cells were prepared. The spleen cells were treated with a HPV6E6 pool peptide (manufactured by JPT Peptide Technologies, catalog # PM-HPV06-E6) or a HPV11E6 pool peptide (manufactured by JPT Peptide Technologies, catalog # PM-HPV11-E6), the culture supernatant after culturing for 48 hours was diluted by 2-fold or 30-fold, and the amount of IFN-γ was measured by a cytokine ELISA method. When the amount of IFN-γ from the 30-fold diluted culture supernatant exceeded the upper limit of detection on the standard curve, a value obtained by multiplying the upper limit by a dilution factor of 30 was taken as data.

[Test Example 3]

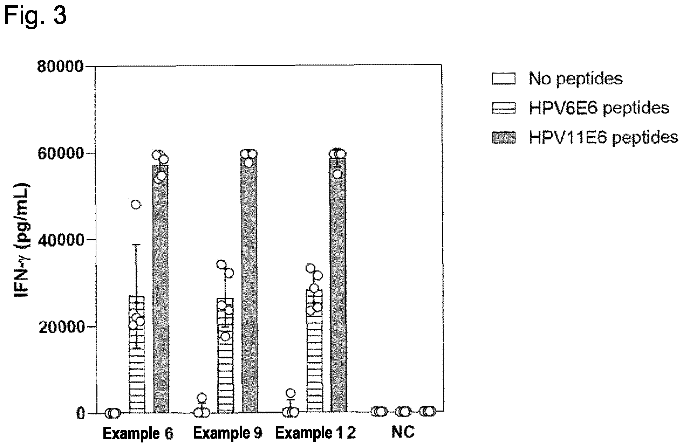

Ability to produce T cell cytokine specific to HPV genotype 6E6E7-genotype 11E6E7 vaccine antigen (Figure 3)

The mRNA-encapsulating nucleic acid lipid particles were administered into the gastrocnemius muscle of six-week-old C57BL/6 mice in an amount of 5 μg of mRNA per mouse twice at an interval of two weeks. One week after the final administration, the spleen was harvested, and spleen cells were prepared. The spleen cells were treated with a HPV6E6 pool peptide (manufactured by JPT Peptide Technologies, catalog # PM-HPV06-E6) or a HPV11E6 pool peptide (manufactured by JPT Peptide Technologies, catalog # PM-HPV11-E6), the culture supernatant after culturing for 48 hours was diluted by 2-fold or 30-fold, and the amount of IFN-γ was measured by a cytokine ELISA method. When the amount of IFN-γ from the 30-fold diluted culture supernatant exceeded the upper limit of detection on the standard curve, a value obtained by multiplying the upper limit by a dilution factor of 30 was taken as data.

[Results of Test Examples 1 to 3]

(Results of Test Example 1)

Level of induction of IFN-γ specific to HPV6E6 and HPV11E6 in Examples 4, 7 and 10 by mRNA-encapsulating nucleic acid lipid particles and having different lipid component ratios

To C57BL/6 mice, mRNA-encapsulating nucleic acid lipid particles in Examples 4, 7 and 10 were intramuscularly administered, and one week after the final immunity, the level of induction of a T cell cytokine specific to HPV6E6 and HPV11E6 from spleen cells was examined. The results are shown in Figure 1. In all groups receiving mRNA-encapsulating nucleic acid lipid particles, due to the HPV6E6 pool peptide treatment, production of IFN-γ was enhanced as compared to that in the NC group. The HPV11E6 pool peptide treatment also led to induction of production of IFN-γ.

(Results of Test Example 2)

Level of induction of IFN-γ specific to HPV6E6 and HPV11E6 in Examples 5, 8 and 11 by mRNA-encapsulating nucleic acid lipid particles and having different lipid component ratios

To C57BL/6 mice, mRNA-encapsulating nucleic acid lipid particles in Examples 5, 8 and 11 were intramuscularly administered, and one week after the final immunization, the level of induction of a T cell cytokine specific to HPV6E6 and HPV11E6 from spleen cells was examined. The results are shown in Figure 2. In all groups receiving mRNA-encapsulating nucleic acid lipid particles, due to the HPV11E6 pool peptide treatment, production of IFN-γ was enhanced as compared to that in the NC group. The HPV6E6 pool peptide treatment also led to induction of production of IFN-γ.

(Results of Test Example 3)

Level of induction of IFN-γ specific to HPV6E6 and HPV11E6 in Examples 6, 9 and 12 by mRNA-encapsulating nucleic acid lipid particles and having different lipid component ratios

To C57BL/6 mice, mRNA-encapsulating nucleic acid lipid particles in Examples 6, 9 and 12 were intramuscularly administered, and one week after the final immunization, the level of induction of a T cell cytokine specific to HPV6E6 and HPV11E6 from spleen cells was examined. The results are shown in Figure 3. In all groups receiving mRNA-encapsulating nucleic acid lipid particles, due to the HPV6E6 pool peptide and HPV11E6 pool peptide treatment, production of IFN-γ was induced as compared to that in the NC group. This indicates that the HPV genotype 6E6E7-genotype 11E6E7 fusion vaccine induced a cellular immune response equivalent to a cellular immune response specific to HPV6E6 in administration of the HPV genotype 6E6E7 vaccine alone and a cellular immune response specific to HPV11E6 in administration of the HPV genotype 11E6E7 vaccine alone.

Industrial Applicability

The present invention can be used for preventing and/or treating infection with human papillomavirus type 6 and/or type 11.

Sequence Listing

SEQ ID NO: 1: template plasmid DNA for IVT of HPV6 E6-E7 fusion
SEQ ID NO: 2: sense primer
SEQ ID NO: 3: antisense primer
SEQ ID NO: 4: HPV6 E6-E7 fusion template DNA
SEQ ID NO: 5: HPV6 E6-E7 fusion mRNA
SEQ ID NO: 6: template plasmid DNA for IVT of HPV11 E6-E7 fusion
SEQ ID NO: 7: HPV11 E6-E7 fusion template DNA
SEQ ID NO: 8: HPV11 E6-E7 fusion mRNA
SEQ ID NO: 9: HPV6 E6-E7 HPV11 E6-E7 fusion template plasmid DNA
SEQ ID NO: 10: HPV6 E6-E7 HPV11 E6-E7 fusion template DNA
SEQ ID NO: 11: HPV6 E6-E7 HPV11 E6-E7 fusion mRNA

SEQ ID NO: 12: amino acid sequence of E6 antigen of HPV type 6
SEQ ID NO: 13: amino acid sequence of E7 antigen of HPV type 6
SEQ ID NO: 14: amino acid sequence of E6 antigen of HPV type 11
SEQ ID NO: 15: amino acid sequence of E7 antigen of HPV type 11
SEQ ID NO: 16: amino acid sequence of protease cleavage sequence
SEQ ID NO: 17: amino acid sequence of fusion protein of E6 antigen and E7 antigen of HPV type 6
SEQ ID NO: 18: amino acid sequence of fusion protein of E6 antigen and E7 antigen of HPV type 11
SEQ ID NO: 19: amino acid sequence of fusion protein of E6 antigen and E7 antigen of HPV type 6 and E6 antigen and E7 antigen of HPV type 11
SEQ ID NO: 20: IgE leader sequence

## Claims

1. A lipid particle encapsulating a nucleic acid capable of expressing an E6 antigen and an E7 antigen of human papillomavirus, wherein a lipid comprises a cationic lipid having the general formula (Ia), or a pharmaceutically acceptable salt thereof:

[Formula 1]

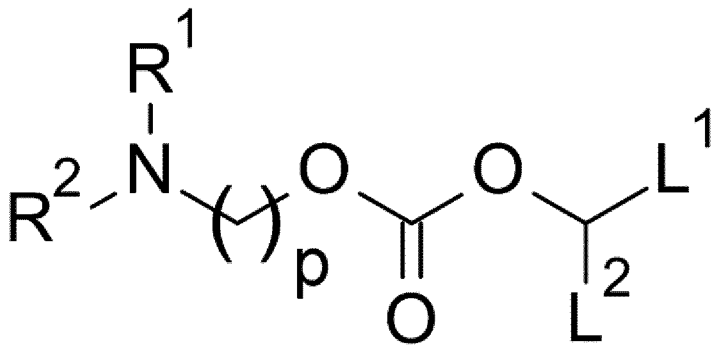

(Ia)

wherein

$R^1$ and $R^2$ each independently represent a C1-C3 alkyl group;
$L^1$ represents a C17-C19 alkenyl group optionally having one or more C2-C4 alkanoyloxy groups;
$L^2$ represents a C10-C19 alkyl group optionally having one or more C2-C4 alkanoyloxy groups, or a C10-C19 alkenyl group, optionally having one or more C2-C4 alkanoyloxy groups; and
p is 3 or 4.

2. The particle according to claim 1, wherein each of $R^1$ and $R^2$ in the general formula (Ia) is a methyl group.

3. The particle according to claim 1 or 2, wherein p in the general formula (Ia) is 3.

4. The particle according to any one of claims 1 to 3, wherein $L^1$ in the genera formula (Ia) is a C17-C19 alkenyl group, optionally having one or more acetoxy groups.

5. The particle according to any one of claims 1 to 4, wherein $L^2$ in the genera formula (Ia) is a C10-C12 alkyl group, optionally having one or more acetoxy groups, or a C10-C19 alkenyl group optionally having one or more acetoxy groups.

6. The particle according to any one of claims 1 to 4, wherein $L^2$ in the genera formula (Ia) is a C10-C12 alkyl group, optionally having one or more acetoxy groups, or a C17-C19 alkenyl group optionally having one or more acetoxy groups.

7. The particle according to any one of claims 1 to 6, wherein $L^1$ in the general formula (Ia) is a (R)-11-acetyloxy-cis-8-heptadecenyl group, a cis-8-heptadecenyl group, or a (8Z,11Z)-heptadecadienyl group.

8. The particle according to any one of claims 1 to 7, wherein $L^2$ in the general formula (Ia) is a decyl group, a cis-7-decenyl group, a dodecyl group, or a (R)-11-acetyloxy-cis-8-heptadecenyl group.

9. The particle according to claim 1, wherein the cationic lipid has the following structural formula:

[Formula 2]

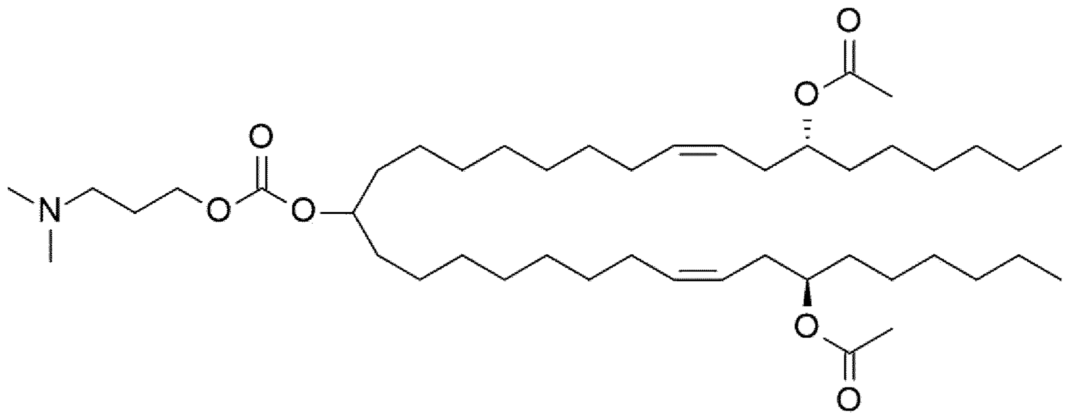

10. The particle according to claim 1, wherein the cationic lipid has the following structural formula:

[Formula 3]

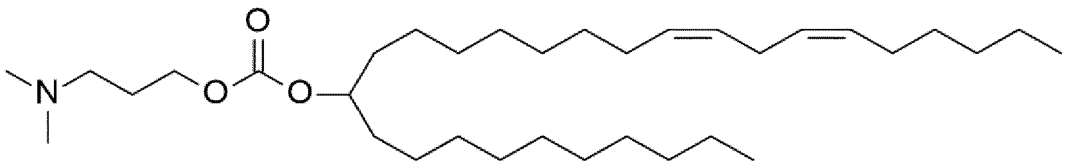

11. The particle according to claim 1, wherein the cationic lipid has the following structural formula:

[Formula 4]

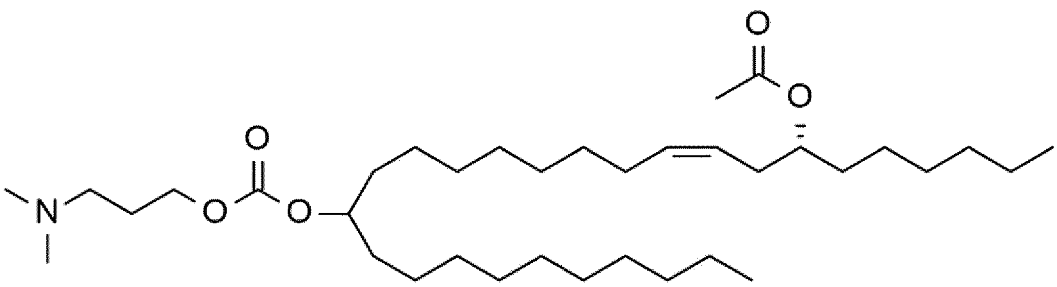

12. The particle according to claim 9 or 10, wherein the lipid further comprises an amphipathic lipid, a sterol and a PEG lipid.

13. The particle according to claim 11, wherein the lipid further comprises an amphipathic lipid, a sterol and a PEG lipid.

14. The particle according to claim 12, wherein the amphipathic lipid is at least one selected from the group consisting of distearoyl phosphatidylcholine, dioleoyl phosphatidylcholine and dioleoyl phosphatidylethanolamine.

**15.** The particle according to claim 13, wherein the amphipathic lipid is at least one selected from the group consisting of distearoyl phosphatidylcholine, dioleoyl phosphatidylcholine and dioleoyl phosphatidylethanolamine.

**16.** The particle according to claim 12 or 14, wherein the sterol is cholesterol.

**17.** The particle according to claim 13 or 15, wherein the sterol is cholesterol.

**18.** The particle according to any one of claims 12, 14 and 16, wherein the PEG lipid is 1,2-dimyristoyl-sn-glycelol methoxypolyethylene glycol and/or N-[methoxypoly(ethylene glycol) 2000]carbamoyl]-1,2-dimyristyloxypropyl-3-amine.

**19.** The particle according to any one of claims 13, 15 and 17, wherein the PEG lipid is 1,2-dimyristoyl-sn-glycelol methoxypolyethylene glycol and/or N-[methoxypoly(ethylene glycol) 2000]carbamoyl]-1,2-dimyristyloxypropyl-3-amine.

**20.** The particle according to any one of claims 12 to 19, wherein the lipid composition of the amphipathic lipid, the sterol, the cationic lipid and the PEG lipid is amphipathic lipid: 5 to 25%, sterol: 10 to 55%, cationic lipid: 40 to 65% and PEG lipid: 1 to 5% on a molar amount basis.

**21.** The particle according to claim 20, wherein the proportion of the amphipathic lipid is 10 to 25%.

**22.** The particle according to any one of claims 12, 14, 16 and 18, wherein the lipid composition of the amphipathic lipid, the sterol, the cationic lipid and the PEG lipid is amphipathic lipid: 5 to 15%, sterol: 35 to 50%, cationic lipid: 40 to 55% and PEG lipid: 1 to 3% on a molar amount basis.

**23.** The particle according to claim 22, wherein the proportions of the amphipathic lipid, the sterol, the cationic lipid and the PEG lipid are 10 to 15%, 35 to 45%, 40 to 50% and 1 to 2.5%, respectively.

**24.** The particle according to claim 23, wherein the proportion of the PEG lipid is 1 to 2%.

**25.** The particle according to any one of claims 13, 15, 17 and 19, wherein the lipid composition of the amphipathic lipid, the sterol, the cationic lipid and the PEG lipid is amphipathic lipid: 10 to 25%, sterol: 10 to 50%, cationic lipid: 40 to 65% and PEG lipid: 1 to 3% on a molar amount basis.

**26.** The particle according to claim 25, wherein the proportions of the sterol, the cationic lipid and the PEG lipid are 10 to 45%, 42.5 to 65% and 1 to 2.5%, respectively.

**27.** The particle according to claim 26, wherein the proportion of the PEG lipid is 1 to 2%.

**28.** The particle according to any one of claims 20 to 27, wherein the ratio of the total weight of lipids to the weight of the nucleic acid is from 15 to 30.

**29.** The particle according to claim 28, wherein the ratio of the total weight of lipids to the weight of the nucleic acid is from 15 to 25.

**30.** The particle according to claim 29, wherein the ratio of the total weight of lipids to the weight of the nucleic acid is from 17.5 to 22.5.

**31.** The particle according to any one of claims 1 to 30, wherein the human papillomavirus is HPV type 6.

**32.** The particle according to claim 31, wherein the human papillomavirus is HPV type 6, and the E6 antigen of HPV type 6 consists of an amino acid sequence having an identity of at least 95% with the amino acid sequence of SEQ ID NO: 12.

**33.** The particle according to claim 31 or 32, wherein the human papillomavirus is HPV type 6, and the E7 antigen of HPV type 6 consists of an amino acid sequence having an identity of at least 95% with the amino acid sequence of SEQ ID NO: 13.

**34.** The particle according to any one of claims 31 to 33, wherein the human papillomavirus is HPV type 6, and the nucleic acid capable of expressing the E6 antigen and the E7 antigen of the human papillomavirus encodes a fusion protein of the E6 antigen and the E7 antigen of HPV type 6, which consists of an amino acid sequence having an identity of at least 95% with the amino acid sequence of SEQ ID NO: 17.

**35.** The particle according to any one of claims 31 to 34, wherein the human papillomavirus is HPV type 6, and the nucleic acid capable of expressing the E6 antigen and the E7 antigen of HPV type 6 is an mRNA comprising a cap structure (Cap), a 5' untranslated region (5'-UTR), a leader sequence, a translated region of E6, a protease cleavage sequence (furin cleavage site), a translated region of E7, a 3' untranslated region (3'-UTR) and a poly A tail (polyA).

**36.** The particle according to claim 35, wherein the sequence of the nucleic acid capable of expressing the E6 antigen and the E7 antigen of HPV type 6 consists of a nucleotide sequence having an identity of at least 90% with the sequence of SEQ ID NO: 5.

**37.** The particle according to any one of claims 31 to 34, wherein the human papillomavirus is HPV type 6, and the nucleic acid capable of expressing the E6 antigen and the E7 antigen of HPV type 6 is an mRNA having a structure comprising a cap structure (Cap), a 5' untranslated region (5'-UTR), a leader sequence, a translated region of E6, a protease cleavage sequence (furin cleavage site), a translated region of E7 and a 3' untranslated region (3'-UTR).

**38.** The particle according to claim 37, wherein the structure comprising a cap structure (Cap), a 5' untranslated region (5'-UTR), a leader sequence, a translated region of E6, a protease cleavage sequence (furin cleavage site), a translated region of E7 and a 3' untranslated region (3'-UTR) consists of a nucleotide sequence having an identity of at least 90% with the sequence of residues 1 to 1018 in SEQ ID NO: 5.

**39.** The particle according to any one of claims 1 to 30, wherein the human papillomavirus is HPV type 11.

**40.** The particle according to claim 39, wherein the human papillomavirus is HPV type 11, and the E6 antigen of HPV type 11 consists of an amino acid sequence having an identity of at least 95% with the amino acid sequence of SEQ ID NO: 14.

**41.** The particle according to claim 39 or 40, wherein the human papillomavirus is HPV type 11, and the E7 antigen of HPV type 11 consists of an amino acid sequence having an identity of at least 95% with the amino acid sequence of SEQ ID NO: 15.

**42.** The particle according to any one of claims 39 to 41, wherein the human papillomavirus is HPV type 11, and the nucleic acid capable of expressing the E6 antigen and the E7 antigen of the human papillomavirus encodes a fusion protein of the E6 antigen and the E7 antigen of HPV type 11, which consists of an amino acid sequence having an identity of at least 95% with the amino acid sequence of SEQ ID NO: 18.

**43.** The particle according to any one of claims 39 to 42, wherein the human papillomavirus is HPV type 11, and the nucleic acid capable of expressing the E6 antigen and the E7 antigen of HPV type 11 is an mRNA comprising a cap structure (Cap), a 5' untranslated region (5'-UTR), a leader sequence, a translated region of E6, a protease cleavage sequence (furin cleavage site), a translated region of E7, a 3' untranslated region (3'-UTR) and a poly A tail (polyA).

**44.** The particle according to claim 43, wherein the sequence of the nucleic acid capable of expressing the E6 antigen and the E7 antigen of HPV type 11 consists of a nucleotide sequence having an identity of at least 90% with the sequence of SEQ ID NO: 8.

**45.** The particle according to any one of claims 39 to 42, wherein the human papillomavirus is HPV type 11, and the nucleic acid capable of expressing the E6 antigen and the E7 antigen of HPV type 11 is an mRNA having a structure comprising a cap structure (Cap), a 5' untranslated region (5'-UTR), a leader sequence, a translated region of E6, a protease cleavage sequence (furin cleavage site), a translated region of E7 and a 3' untranslated region (3'-UTR).

**46.** The particle according to claim 45, wherein the structure comprising a cap structure (Cap), a 5' untranslated region (5'-UTR), a leader sequence, a translated region of E6, a protease cleavage sequence (furin cleavage site), a translated region of E7 and a 3' untranslated region (3'-UTR) consists of a nucleotide sequence having an identity of at least 90% with the sequence of residues 1 to 1018 in SEQ ID NO: 8.

**47.** The particle according to any one of claims 1 to 30, wherein the human papillomavirus is HPV type 6 and type 11.

**48.** The particle according to claim 47, wherein the human papillomavirus is HPV type 6 and type 11, and the nucleic acid capable of expressing the E6 antigen and the E7 antigen of the human papillomavirus encodes a fusion protein of the E6 antigen and the E7 antigen of HPV type 6 and the E6 antigen and the E7 antigen of HPV type 11, which consists of an amino acid sequence having an identity of at least 95% with the amino acid sequence of SEQ ID NO: 19.

**49.** The particle according to claim 47 or 48, wherein the human papillomavirus is HPV type 6 and type 11, and the nucleic acid capable of expressing the E6 antigen and the E7 antigen of HPV type 6 and the E6 antigen and the E7 antigen of HPV type 11 is an mRNA comprising a cap structure (Cap), a 5' untranslated region (5'-UTR), a leader sequence, a translated region of E6 of HPV type 6, a translated region of E7 of HPV type 6, a protease cleavage sequence (furin cleavage site), a translated region of E6 of HPV type 11, a translated region of E7 of HPV type 11, a 3' untranslated region (3'-UTR) and a poly A tail (polyA).

**50.** The particle according to claim 49, wherein the sequence of the nucleic acid capable of expressing the E6 antigen and the E7 antigen of HPV type 6 and the E6 antigen and the E7 antigen of HPV type 11 consists of a nucleotide sequence having an identity of at least 90% with the sequence of SEQ ID NO: 11.

**51.** The particle according to claim 47 or 48, wherein the human papillomavirus is HPV type 6 and type 11, and the nucleic acid capable of expressing the E6 antigen and the E7 antigen of HPV type 6 and the E6 antigen and the E7 antigen of HPV type 11 is an mRNA having a structure comprising a cap structure (Cap), a 5' untranslated region (5'-UTR), a leader sequence, a translated region of E6 of HPV type 6, a translated region of E7 of HPV type 6, a protease cleavage sequence (furin cleavage site), a translated region of E6 of HPV type 11, a translated region of E7 of HPV type 11 and a 3' untranslated region (3'-UTR).

**52.** The particle according to claim 51, wherein the structure comprising a cap structure (Cap), a 5' untranslated region (5'-UTR), a leader sequence, a translated region of E6 of HPV type 6, a translated region of E7 of HPV type 6, a protease cleavage sequence (furin cleavage site), a translated region of E6 of HPV type 11, a translated region of E7 of HPV type 11 and a 3' untranslated region (3'-UTR) consists of a nucleotide sequence having an identity of at least 90% with the sequence of residues 1 to 1798 in SEQ ID NO: 11.

**53.** The particle according to any one of claims 1 to 52, wherein the nucleic acid comprises at least one modified nucleotide.

**54.** The particle according to claim 53, wherein the modified nucleotide comprises at least one of pyrimidine nucleotide substituted at the 5-position and/or pseudouridine optionally substituted at the 1-position.

**55.** The particle according to claim 53, wherein the modified nucleotide comprises at least one selected from the group consisting of 5-methylcytidine, 5-methoxyuridine, 5-methyluridine, pseudouridine and 1-alkylpseudouridine.

**56.** The particle according to claim 53, wherein the modified nucleotide comprises at least one selected from the group consisting of 5-methylcytidine, 5-methyluridine and 1-methylpseudouridine.

**57.** The particle according to any one of claims 1 to 56, which has an average particle size of 30 to 300 nm.

**58.** Use of the particle according to any one of claims 1 to 57, for producing a composition for preventing and/or treating infection with human papillomavirus.

**59.** Use of the particle according to any one of claims 1 to 57, for producing a composition for preventing and/or treating a disease caused by infection with human papillomavirus.

**60.** The use of the particle according to claim 59,
wherein the disease caused by infection with human papillomavirus is recurrent respiratory papillomatosis or condyloma acuminatum.

**61.** The use of the particle according to any one of claims 58 to 60, wherein the infection is caused by human papillomavirus of type 6 or type 11.

**62.** A composition comprising the particle according to any one of claims 1 to 57.

**63.** The composition according to claim 62 for expressing the E6 antigen and the E7 antigen of human papillomavirus *in vivo* or *in vitro.*

**64.** The composition according to claim 62 or 63 for use as a medicament.

**65.** The composition according to claim 64 for inducing an immune reaction against human papillomavirus.

**66.** The composition according to claim 64 or 65 for preventing and/or treating infection with human papillomavirus.

**67.** The composition according to claim 64 or 65 for preventing and/or treating a disease caused by infection with human papillomavirus.

**68.** The composition according to claim 67, wherein the disease caused by infection with human papillomavirus is recurrent respiratory papillomatosis or condyloma acuminatum.

**69.** The composition according to any one of claims 66 to 68, wherein the infection is caused by human papillomavirus of type 6 or type 11.

**70.** A method for expressing an E6 antigen and an E7 antigen of human papillomavirus *in vitro,* comprising introducing the composition according to claim 62 or 63 into cells.

**71.** A method for expressing an E6 antigen and an E7 antigen of human papillomavirus *in vivo,* comprising administering the composition according to any one of claims 62 to 66 to a mammal.

**72.** A method for inducing an immune reaction against human papillomavirus, comprising administering the composition according to claim 64 or 65 to a mammal.

**73.** A method for preventing and/or treating infection with human papillomavirus, comprising administering the composition according to any one of claims 64 to 69 to a mammal.

Fig. 1

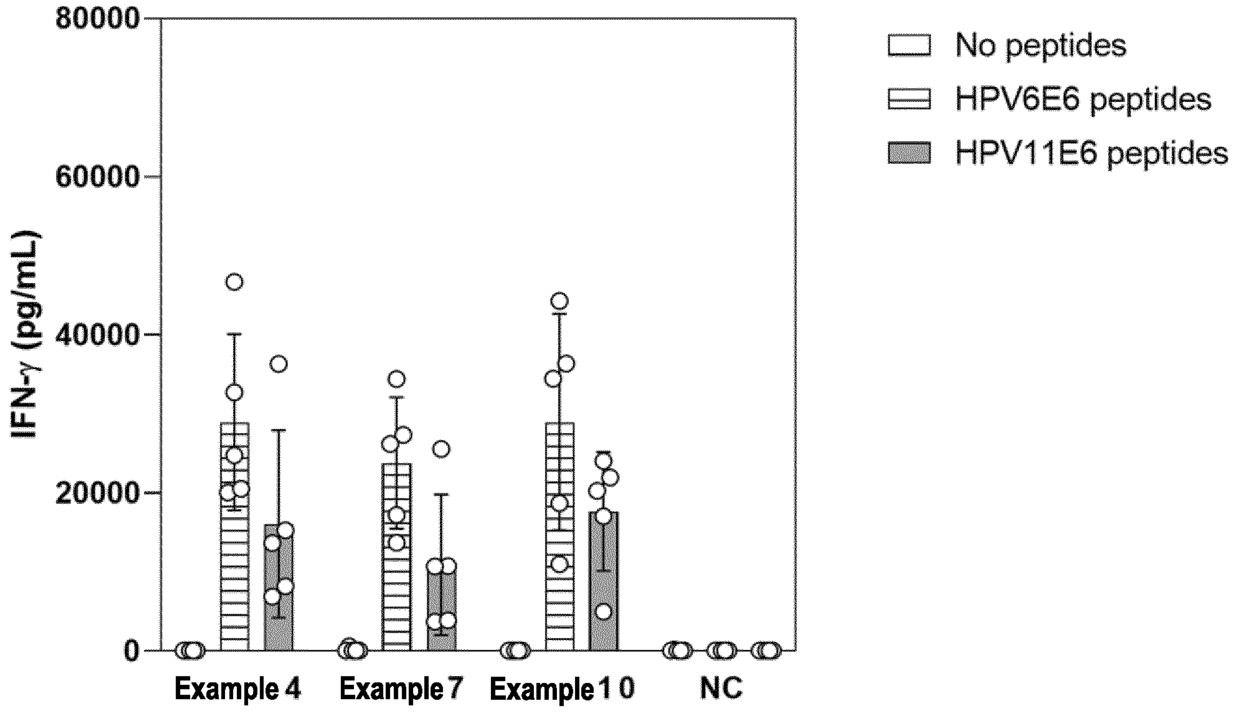
80000
60000
40000
20000
0
IFN-γ (pg/mL)
Example 4
Example 7
Example 1 0
NC
No peptides
HPV6E6 peptides
HPV11E6 peptides

Fig. 2

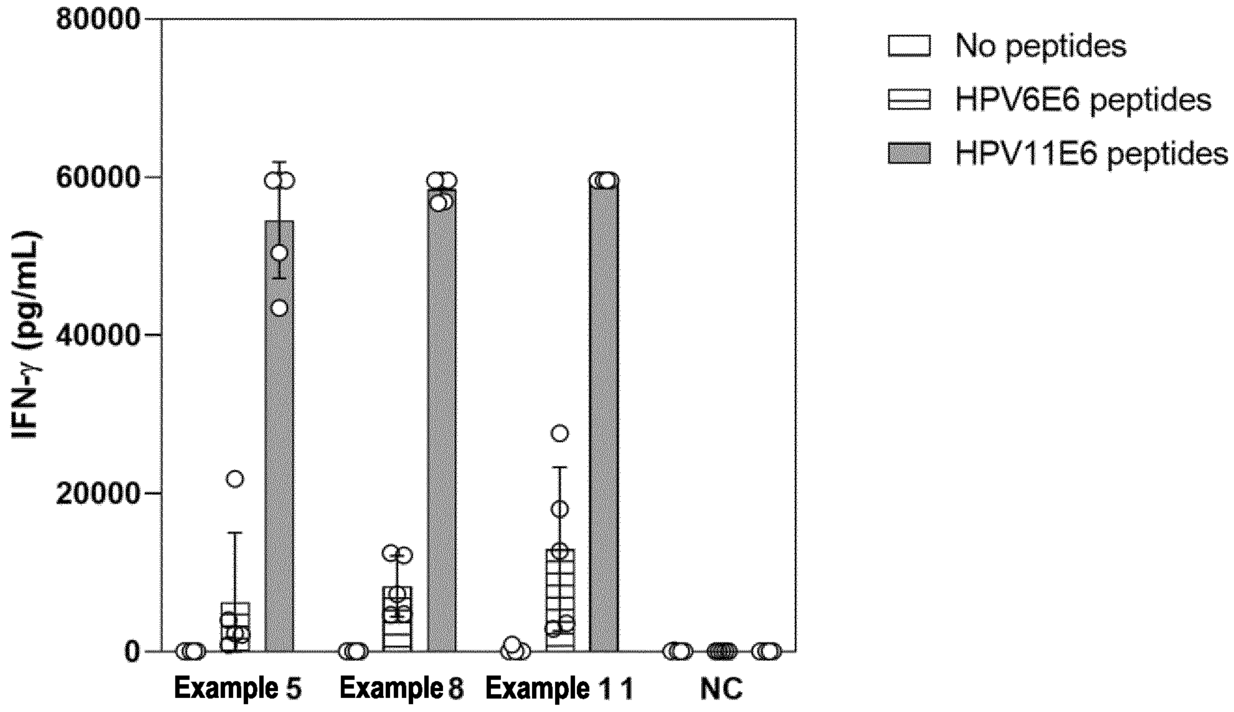
80000
60000
40000
20000
0
IFN-γ (pg/mL)
Example 5
Example 8
Example 1 1
NC
No peptides
HPV6E6 peptides
HPV11E6 peptides

Fig. 3

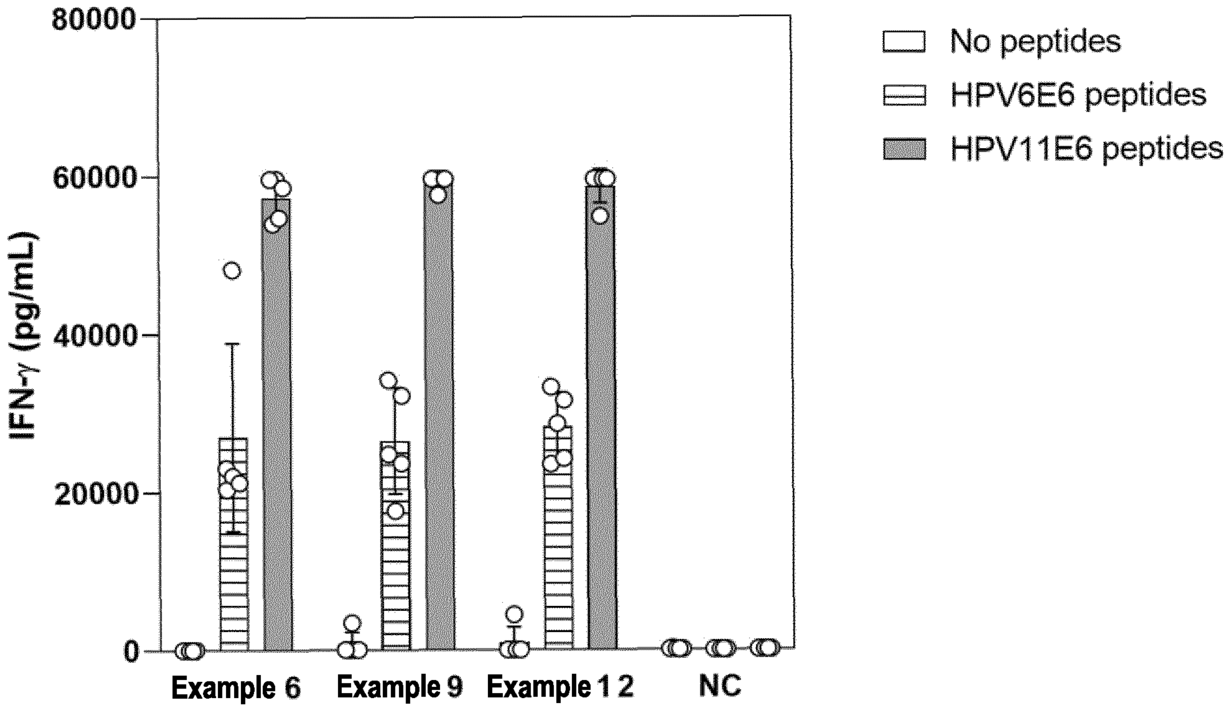
80000
60000
40000
20000
0
IFN-γ (pg/mL)
Example 6
Example 9
Example 1 2
NC
No peptides
HPV6E6 peptides
HPV11E6 peptides

# Fig. 4

## Template plasmid DNA for IVT of HPV6 E6-E7 fusion

gctagcgtaatacgactcactataaggagacccaagctacatttgcttctgacacaactgtgttcactagcaacctcaaacaga
caccgccaccatggactggacctggatcctgttcctggtggccgccgccacaagagtgcacagcgaaagcgccaacgccagc
accagcgccaccaccatcgaccagctgtgcaagaccttcaacctgagcatgcacaccctgcagatcaactgcgtgttctgcaa
gaacgccctgaccaccgccgagatctacagctacgcctacaagcagctgaaggtgctgttcagaggcggctacccctacgcc
gcctgcgcctgcggactggaattccacggcaagatcaaccagtaccggcacttcgactacgccggctacgccaccacagtgg
aagaggaaacaaagcaggacatcctggacgtgctgatccggtgctacctgtgccacaagcccctgtgcgaggtggaaaaagt
gaagcacatcctgaccaaggcccggttcatcaagctgaactgcacctggaaaggcagaggcctgcactgctggaccacctgc
atggaagacatgctgcccagaggccggaagagaagaagccacggcagacacgtgaccctgaaggacatcgtgctggacctg
cagcccccgaccccgtgggactgcacggatacggccagctggtggacagcagcgaggacgaggtggacgaagtggacgg
ccaggacagccagccactgaagcagcactaccagatcgtgacctgctgctgcggctgcgacagcaacgtgcgactggtggtg
cagtgcaccgagacagacatcagagaggtgcagcagctcctgctgggcaccctgaacatcgtgggccccatctgcgcccca
agacctgagctcgctttcttgctgtccaatttctattaaaggttcctttgttccctaagtccaactactaaactgggggatattatg
aagggccttgagcatctggattctgcctaataaaaacatttattttcattgcaaaaaaaaaaaaaaaaaaaaaaaaaaa
aaaaaaaaaaaaaaaaaaaaaaaaaaaaaaaaaaaaaaaaaaaaaaaaaaaaaaaaaaaaaaaaaaaaaaaaaaag
agcactagt
(SEQ ID NO: 1)

GCTAGC (NheI site): base Nos. 1 to 6
T7 promoter sequence: base Nos. 8 to 24
G: Transcription start point: base No. 25
5'-UTR sequence (including transcription start point and KOZAK sequence of base Nos. 89 to 94): base Nos. 25 to 94
Translated region of IgE leader sequence: base Nos. 95 to 148
Translated region of E6 of HPV type 6: base Nos. 149 to 595
Furin cleavage site: base Nos. 596 to 616
Translated region of E7 of HPV type 16: base Nos. 617 to 910
3'-UTR sequence: base Nos. 911 to 1042
polyA: base Nos. 1043 to 1144
ACTAGT (SpeI site): base Nos. 1149 to 1154

Fig. 5

Sense primer

TAATACGACTCACTATAAGGAGAC
(SEQ ID NO: 2)

Antisense primer

TTTTTTTTTTTTTTTTTTTTTTTTTTTTTTTTTTTTTTTTTTTTTTTTTTTTTTTTTTTTTTTTTTTTTTT
TTTTTTTTTTTTTTTTTTTTTTTTTTTTTGCAATGAAAATAAATGTTTTTTATTAGGC
(SEQ ID NO: 3)

# Fig. 6

## HPV6 E6-E7 fusion template DNA

taatacgactcactataaggagacccaagctacatttgcttctgacacaactgtgttcactagcaacctcaaacagacaccgcc
accatggactggacctggatcctgttcctggtggccgccgccacaagagtgcacagcgaaagcgccaacgccagcaccagcg
ccaccaccatcgaccagctgtgcaagaccttcaacctgagcatgcacaccctgcagatcaactgcgtgttctgcaagaacgcc
ctgaccaccgccgagatctacagctacgcctacaagcagctgaaggtgctgttcagaggcggctacccctacgccgcctgcgc
ctgcggactggaattccacggcaagatcaaccagtaccggcacttcgactacgccggctacgccaccacagtggaagaggaa
acaaagcaggacatcctggacgtgctgatccggtgctacctgtgccacaagcccctgtgcgaggtggaaaaagtgaagcaca
tcctgaccaaggcccggttcatcaagctgaactgcacctggaaaggcagaggcctgcactgctggaccacctgcatggaaga
catgctgcccagaggccggaagagaagaagccacggcagacacgtgaccctgaaggacatcgtgctggacctgcagccccc
cgaccccgtgggactgcacggatacggccagctggtggacagcagcgaggacgaggtggacgaagtggacggccaggaca
gccagccactgaagcagcactaccagatcgtgacctgctgctgcggctgcgacagcaacgtgcgactggtggtgcagtgcac
cgagacagacatcagagaggtgcagcagctcctgctgggcaccctgaacatcgtgggccccatctgcgcccccaagacctga
gctcgctttcttgctgtccaatttctattaaaggttcctttgttccctaagtccaactactaaactggggatattatgaagggcctt
gagcatctggattctgcctaataaaaaacatttattttcattgcaaaaaaaaaaaaaaaaaaaaaaaaaaaaaaaaaaaaaa
aaaaaaaaaaaaaaaaaaaaaaaaaaaaaaaaaaaaaaaaaaaaaaaaaaaaaaaaaaaaaaaaaaaa
(SEQ ID NO: 4)

# Fig. 7

## HPV6 E6-E7 fusion mRNA

aggagacccaagcuacauuugcuucugacacaacuguguucacuagcaaccucaaacagacaccgccaccauggac
uggaccuggauccuguuccugguggccgccgccacaagagugcacagcgaaagcgccaacgccagcaccagcgccacc
accaucgaccagcugugcaagaccuucaaccugagcaugcacacccugcagaucaacugcguguucugcaagaacgc
ccugaccaccgccgagaucuacagcuacgccuacaagcagcugaaggugcuguucagaggcggcuaccccuacgccg
ccugcgccugcggacuggaauuccacggcaagaucaaccaguaccggcacuucgacuacgccggcuacgccaccaca
guggaagaggaaacaaagcaggacauccuggacgugcugauccggugcuaccugugccacaagccccugugcgagg
uggaaaaagugaagcacauccugaccaaggcccgguucaucaagcugaacugcaccuggaaaggcagaggccugca
cugcuggaccaccugcauggaagacaugcugcccagaggccggaagagaagaagccacggcagacacgugacccuga
aggacaucgugcuggaccugcagcccccgaccccgugggacugcacggauacggccagcugguggacagcagcgag
gacgagguggacgaaguggacggccaggacagccagccacugaagcagcacuaccagaucgugaccugcugcugcgg
cugcgacagcaacgugcgacugguggugcagugcaccgagacagacaucagagaggugcagcagcuccugcugggca
cccugaacaucgugggccccaucugcgcccccaagaccugagcucgcuuucuugcuguccaauuucuauuaaaggu
uccuuuguucccuaaguccaacuacuaaacuggggauauuaugaagggccuugagcaucuggauucugccuaau
aaaaaacauuuauuuucauugcaaaaaaaaaaaaaaaaaaaaaaaaaaaaaaaaaaaaaaaaaaaaaaaaaaaaaa
aaaaaaaaaaaaaaaaaaaaaaaaaaaaaaaaaaaaaaaaaaaaaaaaaaaaa
(SEQ ID NO: 5)

# Fig. 8

## Template plasmid DNA for IVT of HPV11 E6-E7 fusion

gctagcgtaatacgactcactataaggagacccaagctacatttgcttctgacacaactgtgttcactagcaacctcaaacaga
caccgccaccatggactggacctggatcctgttcctggtggccgccgccacaagagtgcacagcgagagcaaggacgccagc
acaagcgccaccagcatcgaccagctgtgcaagaccttcaacctgagcctgcacaccctgcagatccagtgcgtgttctgcag
aaacgccctgaccaccgccgagatctacgcctacgcctacaagaacctgaaggtcgtgtggcgggacaacttccccttcgccg
cctgcgcctgcggcctggaactccagggaaagatcaaccagtaccggcacttcaactacgccgcctacgcccccacagtgga
agaggaaacaaacgaggacatcctgaaggtgctgatccggtgctacctgtgccacaagcccctgtgcgagatcgagaagctg
aagcacatcctgggcaaagcccggttcatcaagctgaacaaccagtggaaaggcagaggcctgcactgctggaccacctgc
atggaagacctgctgcccagaggccggaagagaagaagccacggcagactggtcaccctgaaggacatcgtgctggacctg
cagcccccgaccccgtgggactgcacggatacggccagctggaagacagcagcgaggacgaggtggacaaagtggacaa
gcaggacgcccagcccctgacacagcactaccagatcctgacctgctgctgcggctgcgacagcaacgtgcggctggtggtg
gaatgcaccgacggcgacatcagacagctgcaggacctgctcctgggcacactgaacatcgtgggccccatctgcgcccca
agccctgagctcgctttcttgctgtccaatttctattaaaggttcctttgttccctaagtccaactactaaactgggggatattatg
aagggccttgagcatctggattctgcctaataaaaaacatttattttcattgcaaaaaaaaaaaaaaaaaaaaaaaaaaaaa
aaaaaaaaaaaaaaaaaaaaaaaaaaaaaaaaaaaaaaaaaaaaaaaaaaaaaaaaaaaaaaaaaaaaaaaaag
agcactagt (SEQ ID NO: 6)

GCTAGC (NheI site): base Nos. 1 to 6
T7 promoter sequence: base Nos. 8 to 24
G: Transcription start point: base No. 25
5'-UTR sequence (including transcription start point and KOZAK sequence of base Nos. 89 to 94): base Nos. 25 to 94
Translated region of IgE leader sequence: base Nos. 95 to 148
Translated region of E6 of HPV type 11: base Nos. 149 to 595
Furin cleavage site: base Nos. 596 to 616
Translated region of E7 of HPV type 11: base Nos. 617 to 910
3'-UTR sequence: base Nos. 911 to 1042
polyA: base Nos. 1043 to 1144
ACTAGT (SpeI site): base Nos. 1148 to 1154

# Fig. 9

## HPV11 E6-E7 fusion template DNA

taatacgactcactataaggagacccaagctacatttgcttctgacacaactgtgttcactagcaacctcaaacagacaccgcc
accatggactggacctggatcctgttcctggtggccgccgccacaagagtgcacagcgagagcaaggacgccagcacaagc
gccaccagcatcgaccagctgtgcaagaccttcaacctgagcctgcacaccctgcagatccagtgcgtgttctgcagaaacgc
cctgaccaccgccgagatctacgcctacgcctacaagaacctgaaggtcgtgtggcgggacaacttccccttcgccgcctgcg
cctgcggcctggaactccagggaaagatcaaccagtaccggcacttcaactacgccgcctacgcccccacagtggaagagga
aacaaacgaggacatcctgaaggtgctgatccggtgctacctgtgccacaagcccctgtgcgagatcgagaagctgaagcac
atcctgggcaaagcccggttcatcaagctgaacaaccagtggaaaggcagaggcctgcactgctggaccacctgcatggaag
acctgctgcccagaggccggaagagaagaagccacggcagactggtcaccctgaaggacatcgtgctggacctgcagcccc
ccgaccccgtgggactgcacggatacggccagctggaagacagcagcgaggacgaggtggacaaagtggacaagcaggac
gcccagcccctgacacagcactaccagatcctgacctgctgctgcggctgcgacagcaacgtgcggctggtggtggaatgcac
cgacggcgacatcagacagctgcaggacctgctcctgggcacactgaacatcgtgggccccatctgcgcccccaagccctga
gctcgctttcttgctgtccaatttctattaaaggttcctttgttccctaagtccaactactaaactgggggatattatgaagggcctt
gagcatctggattctgcctaataaaaaacatttattttcattgcaaaaaaaaaaaaaaaaaaaaaaaaaaaaaaaaaaaaaaa
aaaaaaaaaaaaaaaaaaaaaaaaaaaaaaaaaaaaaaaaaaaaaaaaaaaaaaaaaaaaaaaaaa
(SEQ ID NO: 7)

# Fig. 10

## HPV11 E6-E7 fusion mRNA

aggagacccaagcuacauuugcuucugacacaacuguguucacuagcaaccucaaacagacaccgccaccauggac
uggaccuggauccuguuccugguggccgccgccacaagagugcacagcgagagcaaggacgccagcacaagcgccac
cagcaucgaccagcugugcaagaccuucaaccugagccugcacacccugcagauccagugcguguucugcagaaacg
cccugaccaccgccgagaucuacgccuacgccuacaagaaccugaaggucguguggcgggacaacuuccccuucgcc
gccugcgccugcggccuggaacuccagggaaagaucaaccaguaccggcacuucaacuacgccgccuacgcccccaca
guggaagaggaaacaaacgaggacauccugaaggugcugauccggugcuaccugugccacaagccccugugcgaga
ucgagaagcugaagcacauccugggcaaagcccgguucaucaagcugaacaaccaguggaaaggcagaggccugcac
ugcuggaccaccugcauggaagaccugcugcccagaggccggaagagaagaagccacggcagacuggucacccugaa
ggacaucgugcuggaccugcagcccccgaccccgugggacugcacggauacggccagcuggaagacagcagcgagg
acgagguggacaaaguggacaagcaggacgcccagccccugacacagcacuaccagauccugaccugcugcugcggc
ugcgacagcaacgugcggcuggugguggaaugcaccgacggcgacaucagacagcugcaggaccugcuccugggcac
acugaacaucgugggccccaucugcgcccccaagcccugagcucgcuuucuugcuguccaauuucuauuaaagguu
ccuuuguucccuaaguccaacuacuaaacugggggauauuaugaagggccuugagcaucuggauucugccuaaua
aaaaacauuuauuuucauugcaaaaaaaaaaaaaaaaaaaaaaaaaaaaaaaaaaaaaaaaaaaaaaaaaaa
aaaaaaaaaaaaaaaaaaaaaaaaaaaaaaaaaaaaaaaaaaaaaaaaa
(SEQ ID NO: 8)

# Fig. 11

## HPV6 E6-E7 HPV11 E6-E7 fusion template plasmid DNA

gctagcgtaatacgactcactataaggagacccaagctacatttgcttctgacacaactgtgttcactagcaacctcaaacaga
caccgccaccatggactggacctggatcctgttcctggtggccgccgccacaagagtgcacagcgaaagcgccaacgccagc
accagcgccaccaccatcgaccagctgtgcaagaccttcaacctgagcatgcacaccctgcagatcaactgcgtgttctgcaa
gaacgccctgaccaccgccgagatctacagctacgcctacaagcagctgaaggtgctgttcagaggcggctacccctacgcc
gcctgcgcctgcggactggaattccacggcaagatcaaccagtaccggcacttcgactacgccggctacgccaccacagtgg
aagaggaaacaaagcaggacatcctggacgtgctgatccggtgctacctgtgccacaagcccctgtgcgaggtggaaaaagt
gaagcacatcctgaccaaggcccggttcatcaagctgaactgcacctggaaaggcagaggcctgcactgctggaccacctgc
atggaagacatgctgcccagaggccggaagagaagaagccacggcagacacgtgaccctgaaggacatcgtgctggacctg
cagcccccgaccccgtgggactgcacggatacggccagctggtggacagcagcgaggacgaggtggacgaagtggacgg
ccaggacagccagccactgaagcagcactaccagatcgtgacctgctgctgcggctgcgacagcaacgtgcgactggtggtg
cagtgcaccgagacagacatcagagaggtgcagcagctcctgctgggcaccctgaacatcgtgggccccatctgcgccccca
agaccagaggaagaaagcggagaagcgagagcaaggacgccagcaccagcgccaccagcatcgaccagctctgcaaaac
cttcaacctgagcctgcacacactccagatccagtgcgtgttctgccggaacgccctgacaacagccgaaatctacgcctacgc
ctacaagaacctgaaggtcgtgtggcgggacaacttccccttcgccgcctgcgcctgcggcctcgagctgcagggaaaaatca
accagtacagacacttcaactacgccgcctacgcccccaccgtcgaggaagagacaaacgaggacatcctgaaggtcctcat
ccgctgctacctgtgccacaaacccctctgcgagatcgagaagctgaaacacatcctgggcaaagcccgcttcatcaagctca
acaaccagtggaaaggccggggactccactgctggacaacatgcatggaagacctgctcccccggggcagaaagaggcgc
agccacggaaggctggtcacactgaaagacatcgtcctggacctccagccaccggacccagtcggcctgcacggctacggac
agctcgaggacagcagcgaggacgaagtggacaaggtggacaagcaggacgcccagcccctgacccagcactaccagatc
ctgacatgctgctgcggctgcgacagcaacgtgcggctggtcgtggaatgcaccgacggcgacatcagacagctgcaggacc
tgctcctgggaacactgaacatcgtgggacccatctgcgcccccaagccctgagctcgctttcttgctgtccaatttctattaaag
gttcctttgttccctaagtccaactactaaactgggggatattatgaagggccttgagcatctggattctgcctaataaaaaca
tttattttcattgcaaaaaaaaaaaaaaaaaaaaaaaaaaaaaaaaaaaaaaaaaaaaaaaaaaaaaaaaaaaaaaaa
aaaaaaaaaaaaaaaaaaaaaaaaaaaaaaaaaaaaaagagcactagt
(SEQ ID NO: 9)

GCTAGC (NheI site): base Nos. 1 to 6
T7 promoter sequence: base Nos. 8 to 24
G: Transcription start point: base No. 25
5'-UTR sequence (including transcription start point and KOZAK sequence of base Nos. 89 to 94): base Nos. 25 to 94
Translated region of IgE leader sequence: base Nos. 95 to 148
Translated region of E6 of HPV type 6: base Nos. 149 to 595
Furin cleavage site: base Nos. 596 to 616
Translated region of E7 of HPV type 6: base Nos. 617 to 907
Furin cleavage site: base Nos. 908 to 928
Translated region of E7 of HPV type 11: base Nos. 929 to 1375
Furin cleavage site: base Nos. 1376 to 1396
Translated region of E7 of HPV type 11: base Nos. 1397 to 1690
3'-UTR sequence: base Nos. 1691 to 1822
polyA: base Nos. 1823 to 1924
ACTAGT (SpeI site): base Nos. 1929 to 1934

# Fig. 12

## HPV6 E6-E7 HPV11 E6-E7 fusion template DNA

taatacgactcactataaggagacccaagctacatttgcttctgacacaactgtgttcactagcaacctcaaacagacaccgcc
accatggactggacctggatcctgttcctggtggccgccgccacaagagtgcacagcgaaagcgccaacgccagcaccagcg
ccaccaccatcgaccagctgtgcaagaccttcaacctgagcatgcacaccctgcagatcaactgcgtgttctgcaagaacgcc
ctgaccaccgccgagatctacagctacgcctacaagcagctgaaggtgctgttcagaggcggctacccctacgccgcctgcgc
ctgcggactggaattccacggcaagatcaaccagtaccggcacttcgactacgccggctacgccaccacagtggaagaggaa
acaaagcaggacatcctggacgtgctgatccggtgctacctgtgccacaagcccctgtgcgaggtggaaaaagtgaagcaca
tcctgaccaaggcccggttcatcaagctgaactgcacctggaaaggcagaggcctgcactgctggaccacctgcatggaaga
catgctgcccagaggccggaagagaagaagccacggcagacacgtgaccctgaaggacatcgtgctggacctgcagccccc
cgaccccgtgggactgcacggatacggccagctggtggacagcagcgaggacgaggtggacgaagtggacggccaggaca
gccagccactgaagcagcactaccagatcgtgacctgctgctgcggctgcgacagcaacgtgcgactggtggtgcagtgcac
cgagacagacatcagagaggtgcagcagctcctgctgggcaccctgaacatcgtgggccccatctgcgcccccaagaccaga
ggaagaaagcggagaagcgagagcaaggacgccagcaccagcgccaccagcatcgaccagctctgcaaaaccttcaacct
gagcctgcacacactccagatccagtgcgtgttctgccggaacgccctgacaacagccgaaatctacgcctacgcctacaaga
acctgaaggtcgtgtggcgggacaacttccccttcgccgcctgcgcctgcggcctcgagctgcagggaaaaatcaaccagtac
agacacttcaactacgccgcctacgcccccaccgtcgaggaagagacaaacgaggacatcctgaaggtcctcatccgctgct
acctgtgccacaaacccctctgcgagatcgagaagctgaaacacatcctgggcaaagcccgcttcatcaagctcaacaacca
gtggaaaggccggggactccactgctggacaacatgcatggaagacctgctcccccggggcagaaagaggcgcagccacg
gaaggctggtcacactgaaagacatcgtcctggacctccagccaccggacccagtcggcctgcacggctacggacagctcga
ggacagcagcgaggacgaagtggacaaggtggacaagcaggacgcccagcccctgacccagcactaccagatcctgacat
gctgctgcggctgcgacagcaacgtgcggctggtcgtggaatgcaccgacggcgacatcagacagctgcaggacctgctcct
gggaacactgaacatcgtgggacccatctgcgcccccaagccctgagctcgctttcttgctgtccaatttctattaaaggttcctt
tgttccctaagtccaactactaaactgggggatattatgaagggccttgagcatctggattctgcctaataaaaaacatttatttt
cattgcaaaaaaaaaaaaaaaaaaaaaaaaaaaaaaaaaaaaaaaaaaaaaaaaaaaaaaaaaaaaaaaaaaaaaaa
aaaaaaaaaaaaaaaaaaaaaaaaaaaaaaa

(SEQ ID NO: 10)

# Fig. 13

## HPV6 E6-E7 HPV11 E6-E7 fusion mRNA

aggagacccaagcuacauuugcuucugacacaacuguguucacuagcaaccucaaacagacaccgccaccauggac
uggaccuggauccuguuccugguggccgccgccacaagagugcacagcgaaagcgccaacgccagcaccagcgccacc
accaucgaccagcugugcaagaccuucaaccugagcaugcacacccugcagaucaacugcguguucugcaagaacgc
ccugaccaccgccgagaucuacagcuacgccuacaagcagcugaaggugcuguucagaggcggcuaccccuacgccg
ccugcgccugcggacuggaauuccacggcaagaucaaccaguaccggcacuucgacuacgccggcuacgccaccaca
guggaagaggaaacaaagcaggacauccuggacgugcugauccggugcuaccugugccacaagccccugugcgagg
uggaaaaagugaagcacauccugaccaaggcccgguucaucaagcugaacugcaccuggaaaggcagaggccugca
cugcuggaccaccugcauggaagacaugcugcccagaggccggaagagaagaagccacggcagacacgugacccuga
aggacaucgugcuggaccugcagcccccgaccccgugggacugcacggauacggccagcugguggacagcagcgag
gacgagguggacgaaguggacggccaggacagccagccacugaagcagcacuaccagaucgugaccugcugcugcgg
cugcgacagcaacgugcgacugguggugcagugcaccgagacagacaucagagaggugcagcagcuccugcugggca
cccugaacaucgugggccccaucugcgcccccaagaccagaggaagaaagcggagaagcgagagcaaggacgccagca
ccagcgccaccagcaucgaccagcucugcaaaaccuucaaccugagccugcacacacuccagauccagugcguguuc
ugccggaacgcccugacaacagccgaaaucuacgccuacgccuacaagaaccugaaggucguguggcgggacaacuu
ccccuucgccgccugcgccugcggccucgagcugcagggaaaaaucaaccaguacagacacuucaacuacgccgccu
acgcccccaccgucgaggaagagacaaacgaggacauccugaagguccucauccgcugcuaccugugccacaaaccc
cucugcgagaucgagaagcugaaacacauccugggcaaagcccgcuucaucaagcucaacaaccaguggaaaggccg
gggacuccacugcuggacaacaugcauggaagaccugcucccccggggcagaaagaggcgcagccacggaaggcugg
ucacacugaaagacaucguccuggaccuccagccaccggacccagucggccugcacggcuacggacagcucgaggac
agcagcgaggacgaaguggacaagguggacaagcaggacgcccagccccugacccagcacuaccagauccugacaug
cugcugcggcugcgacagcaacgugcggcuggucguggaaugcaccgacggcgacaucagacagcugcaggaccugc
uccugggaacacugaacaucgugggacccaucugcgcccccaagcccugagcucgcuuucuugcuguccaauuucu
auuaaagguuccuuuguucccuaaguccaacuacuaaacugggggauauuaugaagggccuugagcaucuggau
ucugccuaauaaaaaacauuuauuuucauugcaaaaaaaaaaaaaaaaaaaaaaaaaaaaaaaaaaaaaaaaa
aaaaaaaaaaaaaaaaaaaaaaaaaaaaaaaaaaaaaaaaaaaaaaaaaaaa
(SEQ ID NO: 11)

# Fig. 14

Amino acid sequence of E6 antigen of HPV type 6

ESANASTSATTIDQLCKTFNLSMHTLQINCVFCKNALTTAEIYSYAYKQLKVLFRGGYPYAACACGLEF
HGKINQYRHFDYAGYATTVEEETKQDILDVLIRCYLCHKPLCEVEKVKHILTKARFIKLNCTWKGRGLH
CWTTCMEDMLP
(SEQ ID NO: 12)

# Fig. 15

Amino acid sequence of E7 antigen of HPV type 6

HGRHVTLKDIVLDLQPPDPVGLHGYGQLVDSSEDEVDEVDGQDSQPLKQHYQIVTCCCGCDSNVRL
VVQCTETDIREVQQLLLGTLNIVGPICAPKT
(SEQ ID NO: 13)

# Fig. 16

Amino acid sequence of E6 antigen of HPV type 11

ESKDASTSATSIDQLCKTFNLSLHTLQIQCVFCRNALTTAEIYAYAYKNLKVVWRDNFPFAACACGLEL
QGKINQYRHFNYAAYAPTVEEETNEDILKVLIRCYLCHKPLCEIEKLKHILGKARFIKLNNQWKGRGLH
CWTTCMEDLLP
(SEQ ID NO: 14)

# Fig. 17

Amino acid sequence of E7 antigen of HPV type 11

HGRLVTLKDIVLDLQPPDPVGLHGYGQLEDSSEDEVDKVDKQDAQPLTQHYQILTCCCGCDSNVRLV
VECTDGDIRQLQDLLLGTLNIVGPICAPKP
(SEQ ID NO: 15)

# Fig. 18

Amino acid sequence of protease cleavage sequence

RGRKRRS
(SEQ ID NO: 16)

# Fig. 19

Amino acid sequence of fusion protein of E6 antigen and E7 antigen of HPV type 6

MDWTWILFLVAAATRVHSESANASTSATTIDQLCKTFNLSMHTLQINCVFCKNALTTAEIYSYAYKQL
KVLFRGGYPYAACACGLEFHGKINQYRHFDYAGYATTVEEETKQDILDVLIRCYLCHKPLCEVEKVKHIL
TKARFIKLNCTWKGRGLHCWTTCMEDMLPRGRKRRSHGRHVTLKDIVLDLQPPDPVGLHGYGQLV
DSSEDEVDEVDGQDSQPLKQHYQIVTCCCGCDSNVRLVVQCTETDIREVQQLLLGTLNIVGPICAPK
T
(SEQ ID NO: 17)

# Fig. 20

Amino acid sequence of fusion protein of E6 antigen and E7 antigen of HPV type 11

MDWTWILFLVAAATRVHSESKDASTSATSIDQLCKTFNLSLHTLQIQCVFCRNALTTAEIYAYAYKNLK
VVWRDNFPFAACACGLELQGKINQYRHFNYAAYAPTVEEETNEDILKVLIRCYLCHKPLCEIEKLKHIL
GKARFIKLNNQWKGRGLHCWTTCMEDLLPRGRKRRSHGRLVTLKDIVLDLQPPDPVGLHGYGQLE
DSSEDEVDKVDKQDAQPLTQHYQILTCCCGCDSNVRLVVECTDGDIRQLQDLLLGTLNIVGPICAPKP
(SEQ ID NO: 18)

## Fig. 21

### Amino acid sequence of fusion protein of E6 antigen and E7 antigen of HPV type 6 and E6 antigen and E7 antigen of HPV type 11

MDWTWILFLVAAATRVHSESANASTSATTIDQLCKTFNLSMHTLQINCVFCKNALTTAEIYSYAYKQL
KVLFRGGYPYAACACGLEFHGKINQYRHFDYAGYATTVEEETKQDILDVLIRCYLCHKPLCEVEKVKHIL
TKARFIKLNCTWKGRGLHCWTTCMEDMLPRGRKRRSHGRHVTLKDIVLDLQPPDPVGLHGYGQLV
DSSEDEVDEVDGQDSQPLKQHYQIVTCCCGCDSNVRLVVQCTETDIREVQQLLLGTLNIVGPICAPK
TRGRKRRSESKDASTSATSIDQLCKTFNLSLHTLQIQCVFCRNALTTAEIYAYAYKNLKVVWRDNFPFA
ACACGLELQGKINQYRHFNYAAYAPTVEEETNEDILKVLIRCYLCHKPLCEIEKLKHILGKARFIKLNNQ
WKGRGLHCWTTCMEDLLPRGRKRRSHGRLVTLKDIVLDLQPPDPVGLHGYGQLEDSSEDEVDKVD
KQDAQPLTQHYQILTCCCGCDSNVRLVVECTDGDIRQLQDLLLGTLNIVGPICAPKP

(SEQ ID NO: 19)

## Fig. 22

IgE leader sequence

MDWTWILFLVAAATRVHS
(SEQ ID NO: 20)

## INTERNATIONAL SEARCH REPORT

International application No.
**PCT/JP2022/020713**

**A. CLASSIFICATION OF SUBJECT MATTER**

***C12N 15/88***(2006.01)i; ***A61K 9/10***(2006.01)i; ***A61K 9/14***(2006.01)i; ***A61K 31/7088***(2006.01)i; ***A61K 31/7115***(2006.01)i; ***A61K 39/12***(2006.01)i; ***A61K 47/18***(2006.01)i; ***A61K 47/24***(2006.01)i; ***A61K 47/28***(2006.01)i; ***A61K 47/34***(2017.01)i; ***A61K 48/00***(2006.01)i; ***A61P 11/00***(2006.01)i; ***A61P 15/00***(2006.01)i; ***A61P 31/20***(2006.01)i; ***A61P 37/04***(2006.01)i; ***C12N 15/34***(2006.01)i

FI: C12N15/88 Z ZNA; C12N15/34; A61P31/20; A61P11/00; A61P15/00; A61P37/04; A61K39/12; A61K31/7088; A61K48/00; A61K9/10; A61K9/14; A61K47/18; A61K47/28; A61K47/24; A61K47/34; A61K31/7115

According to International Patent Classification (IPC) or to both national classification and IPC

**B. FIELDS SEARCHED**

Minimum documentation searched (classification system followed by classification symbols)

C12N15/88; A61K9/10; A61K9/14; A61K31/7088; A61K31/7115; A61K39/12; A61K47/18; A61K47/24; A61K47/28; A61K47/34; A61K48/00; A61P11/00; A61P15/00; A61P31/20; A61P37/04; C12N15/34

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN); GenBank/EMBL/DDBJ/GeneSeq; UniProt/GeneSeq

**C. DOCUMENTS CONSIDERED TO BE RELEVANT**

| Category* | Citation of document, with indication, where appropriate, of the relevant passages | Relevant to claim No. |
|---|---|---|
| Y | JP 2019-532657 A (MODERNATX, INC.) 14 November 2019 (2019-11-14) entirety in particular, claims, paragraphs [0027], [0061], [0203]-[0220], [0370], [0418]-[0432], [0537], [0798]-[0802], [1077]-[1079], examples | 1-73 |
| Y | JP 2020-530765 A (GLAXOSMITHKLINE BIOLOGICALS S. A.) 29 October 2020 (2020-10-29) paragraph [0257] | 1-73 |
| Y | JP 2020-172500 A (ARBUTUS BIOPHARMA CORP.) 22 October 2020 (2020-10-22) paragraph [0091] | 1-73 |
| Y | WO 2015/005253 A1 (DAIICHI SANKYO CO., LTD.) 15 January 2015 (2015-01-15) claims, p. 33, example 28 | 1-73 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **11 July 2022** | **19 July 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/020713**

**C. DOCUMENTS CONSIDERED TO BE RELEVANT**

| Category* | Citation of document, with indication, where appropriate, of the relevant passages | Relevant to claim No. |
|---|---|---|
| Y | JP 2020-039341 A (THE TRUSTEES OF THE UNIVERSITY OF PENNSYLVANIA) 19 March 2020 (2020-03-19) claims, paragraphs [0025]-[0032] | 32-34, 36, 38, 40-42, 44, 46, 48, 50, 52 |
| A | JP 2018-531290 A (MODERNATX, INC.) 25 October 2018 (2018-10-25) claims, examples | 1-73 |
| P, X | WO 2021/095838 A1 (DAIICHI SANKYO CO., LTD.) 20 May 2021 (2021-05-20) claims, examples | 1-73 |

Form PCT/ISA/210 (second sheet) (January 2015)

**INTERNATIONAL SEARCH REPORT**

International application No. **PCT/JP2022/020713**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

Form PCT/ISA/210 (continuation of first sheet) (January 2015)

**INTERNATIONAL SEARCH REPORT**
**Information on patent family members**

International application No.
**PCT/JP2022/020713**

| Patent document cited in search report | Publication date (day/month/year) | Patent family member(s) | Publication date (day/month/year) |
|---|---|---|---|
| JP 2019-532657 A | 14 November 2019 | US 2018/0311343 A1<br>claims, paragraphs [0034], [0080], [0315]-[0333], [0490], [0538]-[0552], [0657], [1265]-[1269], [1410], examples<br>WO 2018/081459 A1<br>EP 3532070 A1<br>KR 10-2019-0086681 A<br>CN 110402145 A | |
| JP 2020-530765 A | 29 October 2020 | US 2020/0222526 A1<br>paragraph [0280]<br>WO 2019/016680 A1<br>EP 3655536 A1<br>CN 111108203 A | |
| JP 2020-172500 A | 22 October 2020 | US 2011/0311583 A1<br>paragraph [0123]<br>WO 2010/054384 A1<br>EP 2355658 A1<br>KR 10-2011-0091866 A<br>CN 102281899 A | |
| WO 2015/005253 A1 | 15 January 2015 | US 2016/0257951 A1<br>claims, p. 15, example 28<br>EP 3020701 A1 | |
| JP 2020-039341 A | 19 March 2020 | WO 2013/055326 A2<br>claims, pp. 8, 9<br>EP 2750703 A2<br>CN 103889450 A<br>KR 10-2014-0076613 A | |
| JP 2018-531290 A | 25 October 2018 | US 2018/0289792 A1<br>claims, examples<br>WO 2017/070616 A2<br>EP 3364982 A2<br>CN 108430456 A<br>KR 10-2018-0096591 A | |
| WO 2021/095838 A1 | 20 May 2021 | CN 114650841 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2016512553 A
- WO 2015005253 A


### Non-patent literature cited in the description


- *Virology,* 2013, vol. 445, 2e10
- *J Natl Cancer Inst,* 1995, vol. 87, 796-802
- *Virus Res.,* 2017, vol. 231, 119-127
- *J Clin Virol,* 2005, vol. 32 (1), S7e15
- *Cell,* 1990, vol. 63, 1129e36
- *Cancer Res,* 1996, vol. 56, 4620e4
- *J Virol,* 1989, 2650-2656
- *J Virol,* 1992, 1329-1335
- *J Virol,* 1994, 5698-5705
- *Proc Natl Acad Sci U. S. A,* 2009, vol. 106, 20458e63
- *J Infect Dis.,* 2019, vol. 219 (7), 1016-1025
- *Nat Rev Cancer.,* 2006, vol. 6 (10), 753-763
- *Hum Vaccin Immunother.,* 2012, vol. 8 (4), 470-478
- *J. Biol. Chem.,* 1992, vol. 267, 16396
- *J. Biol. Chem.,* 1991, vol. 266, 12127
- **SAMBROOK, J. et al.** Molecular Cloning a Laboratory Manual. 1989
- **RASHTCHIAN, A.** *Current Opinion in Biotechnology,* 1995, vol. 6 (1), 30-36
- **GIBSON D.G. et al.** *Science,* 2008, vol. 319 (5867), 1215-1220
- **KORMANN, M.** *Nature Biotechnology,* 2011, vol. 29, 154-157